(19)

**Europäisches Patentamt**
**European Patent Office**
**Office européen des brevets**

(11) **EP 3 866 687 B1**

(12) **EUROPEAN PATENT SPECIFICATION**

(45) Date of publication and mention
of the grant of the patent:
**18.09.2024   Bulletin 2024/38**

(21) Application number: **19874016.9**

(22) Date of filing: **17.10.2019**

(51) International Patent Classification (IPC):
*A61B 5/00* (2006.01)   *A61B 5/08* (2006.01)
*A61B 7/00* (2006.01)   *G10L 25/24* (2013.01)
*G10L 25/27* (2013.01)   *G10L 25/66* (2013.01)

(52) Cooperative Patent Classification (CPC):
**A61B 5/4818; A61B 5/7253; A61B 5/7267;**
**A61B 5/7282; A61B 7/003;** G16H 50/70

(86) International application number:
**PCT/AU2019/051135**

(87) International publication number:
**WO 2020/077413 (23.04.2020 Gazette 2020/17)**

(54) **METHOD AND APPARATUS FOR DIAGNOSIS OF MALADIES FROM PATIENT SOUNDS**

VERFAHREN UND VORRICHTUNG ZUR DIAGNOSE VON KRANKHEITEN AUS
PATIENTENGERÄUSCHEN

PROCÉDÉ ET APPAREIL POUR LE DIAGNOSTIC DE MALADIES À PARTIR DE SONS DE PATIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.10.2018  AU 2018903933**

(43) Date of publication of application:
**25.08.2021  Bulletin 2021/34**

(73) Proprietor: **The University of Queensland
St Lucia Brisbane QLD 4067 (AU)**

(72) Inventor: **ABEYRATNE, Udantha
Brisbane, Queensland 4078 (AU)**

(74) Representative: **Barker Brettell LLP
100 Hagley Road
Edgbaston
Birmingham B16 8QQ (GB)**

(56) References cited:
**WO-A2-2018/141013      US-A1- 2012 004 749
US-A1- 2013 184 601      US-A1- 2015 039 110
US-A1- 2015 073 306      US-B2- 10 098 569**

- **BAHOURA ET AL: "Pattern recognition methods applied to respiratory sounds classification into normal and wheeze classes", COMPUTERS IN BIOLOGY AND MEDICINE, NEW YORK, NY, US, vol. 39, no. 9, 1 September 2009 (2009-09-01), pages 824 - 843, XP026558270, ISSN: 0010-4825, [retrieved on 20090724], DOI: 10.1016/J.COMPBIOMED.2009.06.011**
- **BOKOV PLAMEN ET AL: "Wheezing recognition algorithm using recordings of respiratory sounds at the mouth in a pediatric population", COMPUTERS IN BIOLOGY AND MEDICINE, NEW YORK, NY, US, vol. 70, 8 January 2016 (2016-01-08), pages 40 - 50, XP029418978, ISSN: 0010-4825, DOI: 10.1016/J.COMPBIOMED.2016.01.002**
- **KANG BINGBING ET AL: "Snoring and apnea detection based on hybrid neural networks", 2017 INTERNATIONAL CONFERENCE ON ORANGE TECHNOLOGIES (ICOT), IEEE, 8 December 2017 (2017-12-08), pages 57 - 60, XP033345643, DOI: 10.1109/ICOT.2017.8336088**

EP 3 866 687 B1

- **SHI WEN ET AL: "Obstructive Sleep Apnea Detection Using Difference in Feature and Modified Minimum Distance Classifier", 2018 40TH ANNUAL INTERNATIONAL CONFERENCE OF THE IEEE ENGINEERING IN MEDICINE AND BIOLOGY SOCIETY (EMBC), IEEE, 18 July 2018 (2018-07-18), pages 1 - 4, XP033429094, DOI: 10.1109/EMBC.2018.8513093**
- **DULIP L HERATH ET AL: "Hidden Markov modelling of intra-snore episode behavior of acoustic characteristics of obstructive sleep apnea patients", PHYSIOLOGICAL MEASUREMENT, INSTITUTE OF PHYSICS PUBLISHING, BRISTOL, GB, vol. 36, no. 12, 26 October 2015 (2015-10-26), pages 2379 - 2404, XP020292914, ISSN: 0967-3334, [retrieved on 20151026], DOI: 10.1088/0967-3334/36/12/2379**

**Description**

**TECHNICAL FIELD**

The present invention concerns a method and automated apparatus for diagnosing maladies of the respiratory system from patient sounds.

**RELATED APPLICATIONS**

The present application claims priority from Australian provisional patent application No. 2018903933 filed 17 October 2018.

**BACKGROUND ART**

Any references to methods, apparatus or documents of the prior art are not to be taken as constituting any evidence or admission that they formed, or form part of the common general knowledge.

One common malady is the sleep disorder of Obstructive Sleep Apnea syndrome (OSA). The prevalence of OSA in adults varies from 17-26% in males and 9-28% in females [2]. At present over 85% of OSA patients remain undiagnosed [3]. OSA is characterized by a repetitive upper airway collapse during sleep. Full closure of the upper airway is termed "apnea" and partial closure is termed "hypopnea". The average number of apnea and hypopnea events per-hour of sleep is termed the Apnea-Hypopnea Index (AHI). AHI is a major clinical severity measure for OSA.

The current standard for OSA diagnosis is Polysomnography (PSG)[4]. PSG requires continuous monitoring of multiple physiological signals over the course of a night. Physical contact of sensors with the patient is essential for these measurements. The several hours of PSG data are manually reviewed by an expert sleep technician. Reviewing PSG data is a labor intensive, time consuming and expensive process. PSG is also inconvenient to patients, especially the pediatric population, and results are subjective and unsuitable for population screening.

In the past several researchers have attempted to use patient sounds for diagnosis of maladies related to dysfunctions of the respiratory system. For example, the patient sounds may include snoring sounds used to diagnose OSA. Other maladies, such as pneumonia, asthma, bronchitis, croup and chronic obstructive pulmonary disease (COPD), Tracheobronchomalacia (TBM) or cystic fibrosis also cause characteristic patient sounds. Many of the existing methods depend on the identification of segments of the patient sound that are characteristic of the malady in question. For example, in the case of the malady being OSA then snore segments from the overnight sound data are identified. Hence if the snore segmentation algorithm fails to identify any snore segments or if the patient did not snore then results of the test will be indeterminate. Furthermore, procedures for identifying sounds that are characteristic of a malady of interest, such as snore sounds for OSA diagnosis, or a cough sound for pneumonia diagnosis, in a lengthy patient sound recording are computationally expensive and may be inaccurate. Therefore there is a need for an improved method of diagnosing a malady which does not rely on identification of sounds that are characteristic of a malady of interest in segments of the patient sounds.

US 2012/004749 A1 discloses an apparatus for diagnosing sleep disorders such as OASHS from snore sounds. The apparatus includes a segmentation module coupled to a data logger to provide segments of the digitized audio signal to a Snore Segment Identifier. A total airways response (TAR) module, pitch calculator and MFCC calculator are each coupled to an output side of the snore segment identifier module. Each of these modules is respectively arranged to calculate pitch, bispectrum, diagonal slice and MFCC parameters for the snore segments received from the snore segment identifier (128). Similarly, the NGI calculator produces a non-Gaussianity index for the digitized audio signal. A classification module is arranged to process the calculated parameters and compare a resulting diagnosis probability to a predetermined threshold value. The results of this comparison are then indicated on video display, which communicates with the classification module via display controller and bus. For example, if the results of the comparison are over threshold then display is driven to indicate "OS AHS is present".

US 10,098,569 B2 discloses a method of operating a computational device to process patient sounds. The method comprises the steps of: extracting features from segments of said patient sounds; and classifying the segments as cough or non-cough sounds based upon the extracted features and predetermined criteria; and presenting a diagnosis of a disease related state on a display under control of the computational device based on segments of the patient sounds classified as cough sounds.

**SUMMARY OF THE INVENTION**

According to a first aspect of the present invention there is provided a method of operating one or more electronic processors to diagnose a maladay of the respiratory system of a patient comprising:

accessing with said processors a digital audio signal of sounds of the patient in an electronic storage assembly;
identifying a number of epochs of the digital audio signal;
identifying a plurality of sub-segments for each of the epochs;
for each sub-segment of each of the epochs determining an associated multiplicity of mel-frequency cepstral coefficients (MFCCs);
determining deviation scores from a probability distribution for each of the epochs in respect of each of the multiplicity of MFCCs;
forming a test vector for the patient based upon the deviations scores from the probability distribution of the MFCCs;
applying the test vector to a pre-trained decision machine stored in said electronic storage assembly to thereby generate a malady signal indicating malady or non-malady for the patient; and
controlling a display responsive to the one or more electronic processors to display a message corresponding to the malady signal.

According to a preferred embodiment of the present invention the forming of the test vector based upon the deviations scores of the MFCCs includes applying a comparator to each of the deviation scores. For example, the comparator may comprise a set of instructions executed by the one or more processors to implement a decision routine.

In an embodiment the output of the routine is a "1" signal if the deviation score is above a threshold or a "0" signal if the deviation score is equal to or below the threshold.

Preferably the method further includes forming components of the test vector for each of the MFCCs by producing sums of outputs from the comparator. In an embodiment the method includes producing the sums of the outputs from the comparator for each MFCC over all of the epochs.

The method may include averaging each of the sums of the outputs over all of the epochs.

In a preferred embodiment of the invention the method includes reducing dimensionality of the test vector. For example, the method may include removing all but a subset of components of the test vector previously adjudged to be statistically significant for production of the malady signal from the pre-trained decision machine.

Preferably the method includes forming the test vector on the basis of the entire digital audio signal.

In one embodiment of the invention the probability distribution is a Gaussian distribution and the deviation from a probability distribution score is a non-Gaussianity Score (NGS) or non-Gaussianity "Index" though other distributions may also be used and measures of deviation from those distributions may also be used.

For example, other embodiments may involve computing a KS test (Kolmogorov-Smirnov) test statistic in the place of the Chi-squared test statistic.

Another embodiment of the invention may make use of a Lilliefors test for normalcy with the Gaussian distribution.

The malady may be OSA.

According to a second aspect of the present invention there is provided an apparatus for diagnosing the presence of a malady of the respiratory system of a patient comprising:

a microphone;
an audio interface including an analog-to-digital converter (ADC) coupled to the microphone;
an electronic storage assembly coupled to the ADC and arranged to store a digitized audio file of patient sounds from the audio interface;
an epoch identification assembly configured to process the digitized audio file and identify a number of epochs therein;
a sub-segment identification assembly configured to process the digitized audio file and identify a plurality of sub-segments therein for each of the epochs;
a Mel-Frequency Cepstral Coefficient generator that is responsive to the epoch identification assembly and the sub-segment identification assembly and arranged to process the digitized audio file to produce a multiplicity of mel-frequency cepstral coefficients (MFCCs) signals for each of the sub-segments;
a deviation from probability distribution score assembly that is responsive to the Mel-Frequency Cepstral Coefficient generator and which is arranged to process the MFCCs signals for each of the sub-segments to produce deviation from probability distribution scores for each of the MFCCs signals for each epoch;
a test-vector generator assembly that is responsive to the deviation from probability distribution score assembly and which is arranged to store a test vector for the patient in the electronic storage assembly;
a decision assembly that is coupled to the at least one electronic processor and arranged to process the test vector to produce a OSA diagnosis signal; and
a human-machine interface that is coupled to the decision assembly and arranged to present the OSA diagnosis to a human.

The malady may be OSA.

According to a third aspect, there is provided a computer readable medium bearing tangible, non-transitory machine readable instructions for execution by one or more electronic microprocessors including instructions for performing the method of the first aspect.

The distribution may be a Gaussian distribution and the deviation from probability distribution score assembly is a non-Gaussianity score (NGS) assembly and the deviation score is a non-Gaussianity Score or "index". It will be realized that other distributions are also useable and some of these other distributions are described toward the end of this specification.

To aid understanding of the invention, a method for diagnosing a malady of a patient from sounds of the patient is disclosed, that is not presently covered by the claims as granted. The method includes the steps of:

> making a digital recording of the sounds of the patient;
> processing the digital recording to extract one or more features for sub-segments of each of a number epochs of the digital recording;
> determining deviation scores from a probability distribution for each epoch based on said extracted features;
> applying a test vector derived from the deviation scores to a pre-trained decision machine; and
> presenting a diagnosis of the malady based on an output from said decision machine.

For example, the malady may comprise OSA or a disease state such as pneumonia or another malady that causes a change from normal patient sounds, such as, pneumonia, asthma, bronchitis, croup and chronic obstructive pulmonary disease (COPD), Tracheobronchomalacia (TBM) or cystic fibrosis.

The features may be one or more of pitch, entropy, formants, a Gaussianity or other probability distribution measure and higher-order spectra-based features.

The method may involve computing a Chi-squared test statistic between a MFCC distribution and a target probability distribution and using the computed test statistic directly as a feature to input to the decision machine.

The method may alternatively involve computing p-values for a Chi-squared test statistic between a MFCC distribution and the target distribution and use the p-value directly as a feature to feed the decision machine.

The target distribution may be a Gaussian distribution.

Alternatively, the method may involve computing a KS test (Kolmogorov-Smirnov) test statistic in the place of the Chi-squared test statistic.

The method may make use of a Lilliefors test for normalcy with the Gaussian distribution.

To further aid understanding, there is also provided a method for diagnosing OSA of a patient, that is not presently covered by the claims as granted. The method for diagnosing OSA of a patient includes the steps of:

> making a digital recording of sounds of the patient;
> processing the digital recording to extract a multiplicity of MFCCs for sub-segments of each of a number epochs of the digital recording;
> determining deviation scores from a probability distribution for each epoch based on the MFCCs;
> applying a test vector derived from the deviation scores to a pre-trained decision machine; and
> presenting a diagnosis of OSA on the basis of an output from said decision machine.

An additional method for diagnosing OSA of a patient is also provided, that is not present covered by the claims as granted, the additional method including the steps of:

> making a digital recording of sounds of the patient;
> processing the digital recording to extract a multiplicity of MFCCs for sub-segments of each of a number epochs of the digital recording;
> determining deviation from a probability distribution score for each epoch based on the MFCCs;
> applying a test vector derived from the deviation from probability distribution score to a pre-trained decision machine; and
> presenting a diagnosis of OSA on the basis of an output from said decision machine.

## BRIEF DESCRIPTION OF THE DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of the Invention in any way. The Detailed Description will make reference to a number of drawings as follows:

Figure 1.    is a block diagram of an example of an example of a specially configured diagnostic device for diagnosing a malady such as OSA, according to a preferred embodiment of the present invention.

Figure 2:      Is a physical view of the diagnostic device of Figure 1 displaying a recording duration and sampling rate screen on its LCD touch screen interface.

Figure 3:      Is a physical view of the diagnostic device of Figure 1 displaying a recording-in-progress screen on its LCD touch screen interface.

Figure 4        Is a first portion of a flowchart of a method coded as instructions in a diagnostic App stored in a digital memory of the diagnostic device.

Figure 5        is a second portion of the flowchart of Figure 4.

Figure 6        depicts a portion of a digital waveform of a patient sound generated by an analog-to-digital converter of the diagnostic device.

Figure 7        depicts a longer duration of the digital waveform showing a number of epochs identified therealong.

Figure 8        depicts a single epoch of the digital waveform with pre-emphasis applied and showing sub-segments of the epoch identified therealong.

Figure 9        is a flowchart of a procedure that is implemented by the diagnostic device during its operation for generating MFCCs for each subsegment.

Figure 10       graphically illustrates MFCCs for each sub-segment of each pre-emphasized epoch.

Figure 11       graphically illustrates the values of three exemplary MFCCs each over a single pre-emphasized epoch.

Figure 12       comprises three data plots of sample distributions that are referred to for explanation of non-Gaussianity score.

Figure 13       graphically illustrates the non-Gaussianity of each of the three data plots and presents a Non-Gaussianity Score (NGS) for each.

Figure 14       graphically illustrates non-Gaussianity Scores for each of three MFCCs for each of N pre-emphasized epochs.

Figure 14A      Is a physical view of the diagnostic device of Figure 1 displaying a diagnosis on its LCD touch screen interface.

Figure 15:      Comprises three charts of Mean (a) Age (b) Body Mass Index (BMI) and (c) Neck Circumference (NC) with 95% confidence interval across the 4 OSA subject groups.

Figure 16:      Is a boxplot showing classification performance of the LRM when trained using features computed with different audio file format. The tops and bottoms of each "box" are the 25th and 75th percentiles of the samples, respectively. Error bar indicates interquartile ranges. The line in the middle of each box is the sample median.

Figure 17:      Is a chart of test classification results of the LRM with change in sampling rate of the audio data. Only selected features were used to train and test the models.

Figure 18:      Illustrates Mean classification Sensitivity and Specificity of LRM with 95% confidence interval, when trained using features computed with data sampled at different sampling rate (Fs). There is no significance difference in LRM sensitivity at different FS. Only specificity of LRM at Fs = 2000 Hz is significantly lower from that at Fs = 44100.

Figure 19       is a block diagram of a dedicated diagnosis apparatus according to a further embodiment of the present invention.

**DETAILED DESCRIPTION OF PREFERRED EMBODIMENTS**

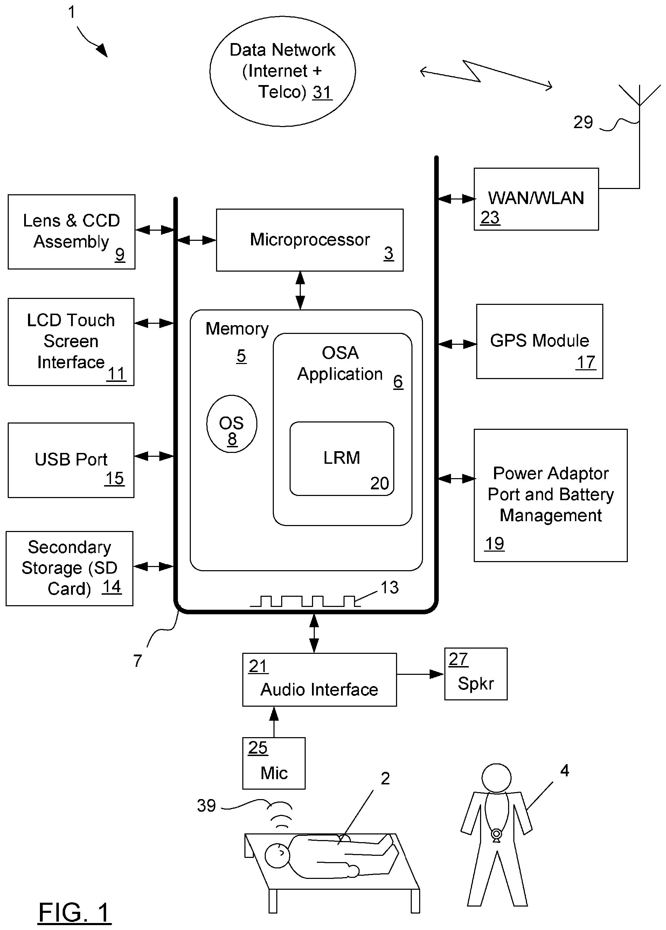

Referring initially to Figure 1 there is shown a diagnostic device 1 in the form of a unique combination being a computational device in the form of a smart phone in combination with a diagnostic application software product. The diagnostic device 1 includes at least one microprocessor 3 that accesses an electronic memory 5. The electronic memory 5 includes an operating system 8 such as the *Android* operating system or the *Apple iOS* operating system, for example, for execution by the microprocessor 3. The electronic memory 5 also includes the diagnostic application software product or "App" 6 according to a preferred embodiment of the present invention. The diagnostic App 6 includes instructions that are executable by the microprocessor 3 in order for the diagnostic device 1 to process sounds from a patient 2 and present a diagnosis of a malady such as OSA to a clinician 4 by means of LCD touch screen interface 11. In the exemplary embodiment that will be primarily discussed reference will be made to a malady being OSA and thus the device will be referred to as OSA diagnostic device 1. However in other embodiments the device 1 may be configured by App 6 to diagnose other maladies of the respiratory system such as pneumonia, asthma, bronchitis, croup and chronic obstructive pulmonary disease (COPD), Tracheobronchomalacia (TBM) or cystic fibrosis. The App 6 includes instructions for the microprocessor 3 to implement a trained predictor or decision machine, which in the presently described preferred embodiment of the invention comprises a specially trained Logistic Regression Model 20. It will be realised that in other embodiments of the invention other suitable decision machines may be used, such as an artificial neural network or a Bayesian decision machine and thus the invention is not limited to the use of an LRM only.

The microprocessor 3 is in data communication with a plurality of peripheral assemblies 9 to 23, as indicated in Figure 1, via a data bus 7. Consequently, if required the diagnostic device 1 is able to establish voice and data communication with a voice and/or data communications network 31 via WAN/WLAN assembly 23 and radio frequency antenna 29.

Although the OSA diagnostic device 1 that is illustrated in Figure 1 is provided in the form of a smartphone it might equally be some other computational device such as a laptop, or tablet in combination with a software product containing instructions to implement a method according to an embodiment of the invention such as will be described.

In a preferred embodiment the OSA diagnostic device 1 is programmed with App 6 so that it operates as a decision device that requires no external sensors, physical contact with patient 2 or communication network 31.

In use the nominal distance from the microphone 25 of device 1 to the face of patient 2 is set to about 50cm, but may vary between 40cm to 70cm due to patient movements.

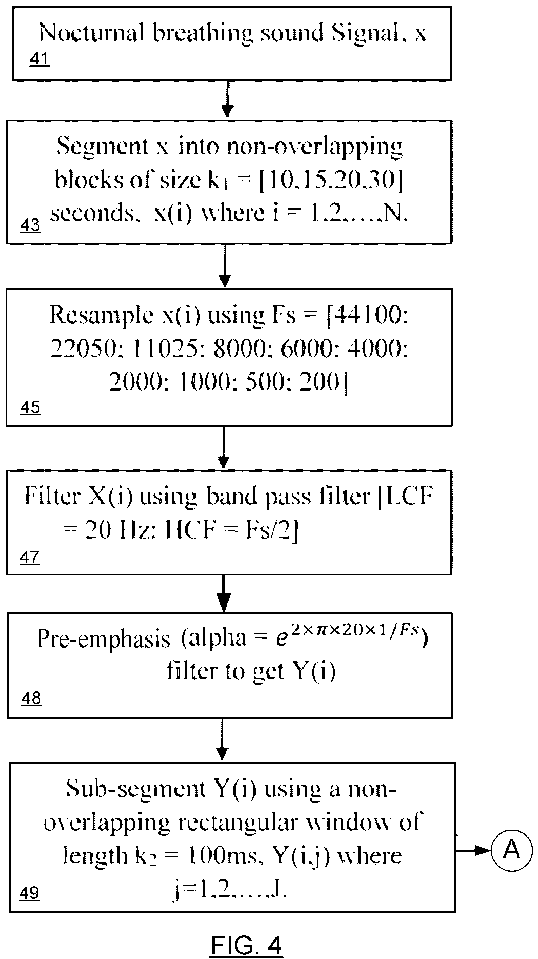

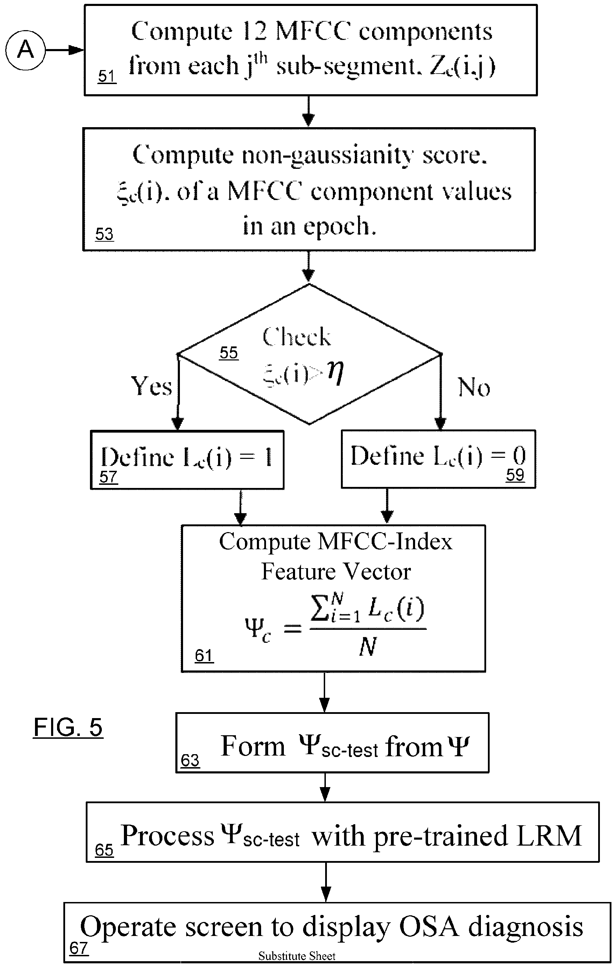

Referring now to Figures 4 and 5, there is shown a flowchart of a method according to a preferred embodiment of the present invention, which the OSA diagnosis device 1 implements under the control of the instructions that are coded into the OSA diagnostic App 6 in order to make a diagnosis of whether or not patient 2 is suffering from a malady, being OSA in the present exemplary embodiment. The health carer 4 can then use the diagnosis to provide appropriate therapy, for example a positive pressure airway device or other suitable therapy to alleviate the OSA.

At box 41 of Figure 4, the microprocessor 3 operates the LCD screen 11 to display a prompt for a user, e.g. clinician 4, to commence recording the in-air sounds 39 of patient 2.

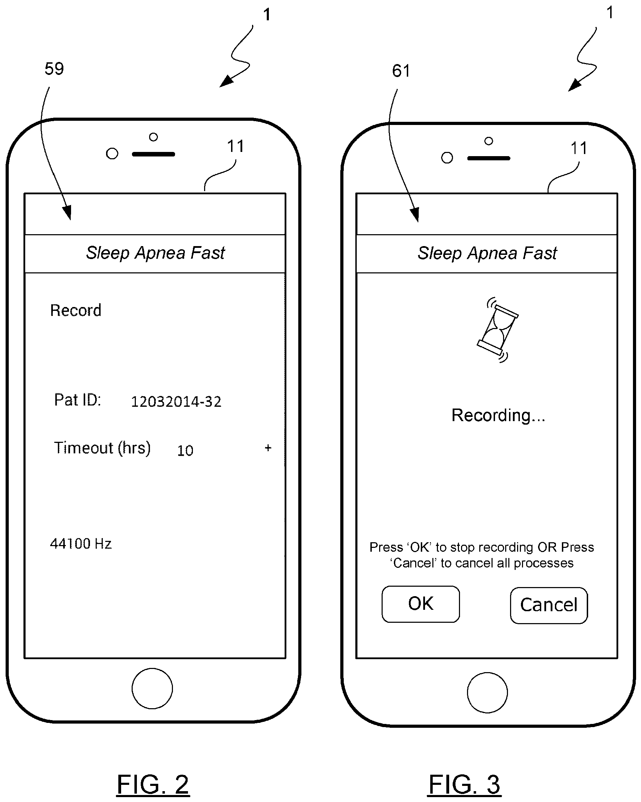

The breathing sound 39 of patient 2 is recorded by the diagnostic device 1 and Figure 2 shows the diagnostic device 1 displaying a recording commencement screen 59 on LCD touch screen 11 for the clinician 4 to enter the recording parameters. The recording parameters are a patient ID number, and the "Timeout", i.e. the duration of the recording that is to be made and also the analogue to digital sample rate to be used. In the present instance the duration that has been selected is 10 hours and the sample rate that is to be used is 44.1 kHz. Figure 3 shows the screen 61 that is displayed once clinician 4 presses the "Record" button in screen 59 of Figure 2.

As the recording proceeds an audio file is stored in an electronic storage assembly such as either memory 5 or secondary memory 14, which is typically a Secure Digital (SD) memory card. The audio file may be stored in a compressed format such as MP3 or in a non-compressed format such as a WAV or FLAC file. The pros and cons of using a compressed format as opposed to an uncompressed format will be discussed later in this specification. Depending on the hardware configuration the selection of the sample rate may alter a sample rate parameter in Audio interface 21 or alternatively the analog-to-digital conversion may be made at 44.1 kHz in the audio interface 21 and then down-sampled by the microprocessor 3 in accordance with instructions in OSA Application 6.

The procedure that microprocessor 3 uses to make a diagnosis of a malady, which in the present example is OSA, and which comprises instructions that make up App 6 is illustrated in the flowchart of Figures 4 and 5 which will now be further described.

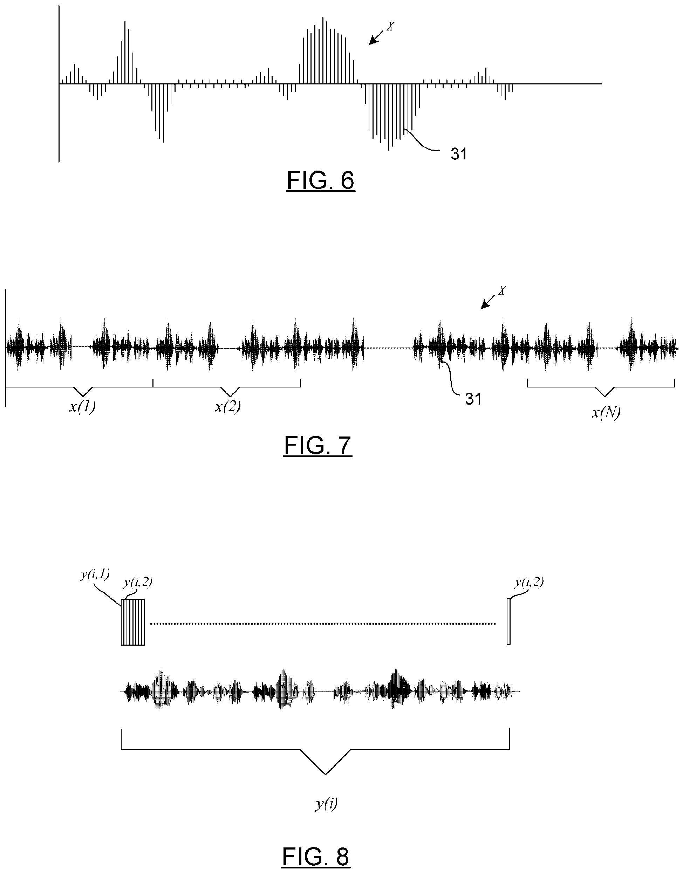

1. At box 41 the nocturnal breathing sound signal 39 of the patient 2 is recorded by microphone 25 and digitized by the audio interface 21 to produce a digitized signal "x" (indicated as item 31 of Figure 6) that is conveyed to microprocessor 3 along bus 7. The digitized patient sound data is recorded from patient 2 at sampling frequency Fs which is typically 44.1 kHz or less.

Figure 7, which shows a far longer portion of the waveform *x* on a much compressed timescale (horizontal axis) than in Figure 6. At box 43 (Figure 4) the microprocessor 3 segments signal *x* into non-overlapping blocks of size $k_1$ = [10, 15, 20, 30] seconds. In a clinical setting these non-overlapping blocks are referred as 'epochs' and thus from here onward the same terminology will be followed. There are a total of N epochs as indicated in Figure 7 and the symbol *x(i)* represents the $i^{th}$ epoch, where *i* = 1,2,...,N.

2. In the present example the OSA diagnosis device 1 has been set to record the patient sound 39 at Fs = 44100 Hz. If at box 45 a lower sampling frequency is desired, for example to decrease the size of the resulting file, then microprocessor 3 can execute instructions in OSA App 6 to resample *x(i)* by choosing sampling frequency from one of (for example) Fs = [22050; 11025; 8000; 6000; 4000; 2000; 1000; 500; 200;] Hz.

3. At box 47 the microprocessor filters each of the epochs *x(1)*,...,*x*(N) using a Butterworth band-pass filter with lower cut-off frequencies (LCF) = 20 Hz (to remove low frequency rumble) and higher cut-off frequency (HCF) = Fs/2.

4. At box 48 microprocessor 3 applies a pre-emphasis filter, as set out in equation (1) below to produce pre-emphasized epochs *y(i)*. If *a[p]* represents signal *x(i)* in $i^{th}$ epoch with *p* = 1,2...,P as sample numbers then a pre-emphasis filter can be implemented as shown in (1). The pre-emphasis filtering boosts the overall energy of the audio signal and improves the signal-to-noise ratio at higher frequencies. The parameter $\alpha$ in (1) can be adjusted to set the filter to the desired cut-off frequency using Fc in (2). The App 6 can include instructions for the microprocessor 3 to present controls on the touchscreen of device 1 for the user to make adjustments if required.

$$b[p] = a[p] - \alpha a[p-1] \qquad (1)$$

$$\alpha = e^{-2\pi \frac{F_c}{F_s}} \qquad (2)$$

5. At box 49 microprocessor 3 sub-segments each of the pre-emphasis filtered epochs *y(i)* using a non-overlapping rectangular window of length $k_2$ = 100 ms. Let y(*i,j*) represent $j^{th}$ sub-segment of the $i^{th}$ epoch data. *j* = 1,2,...,J as illustrated in Figure 8. For example, if the Epoch length $k_1$ is 10 seconds then there will be 10,000 subsegments in each epoch and thus J will equal 10,000.

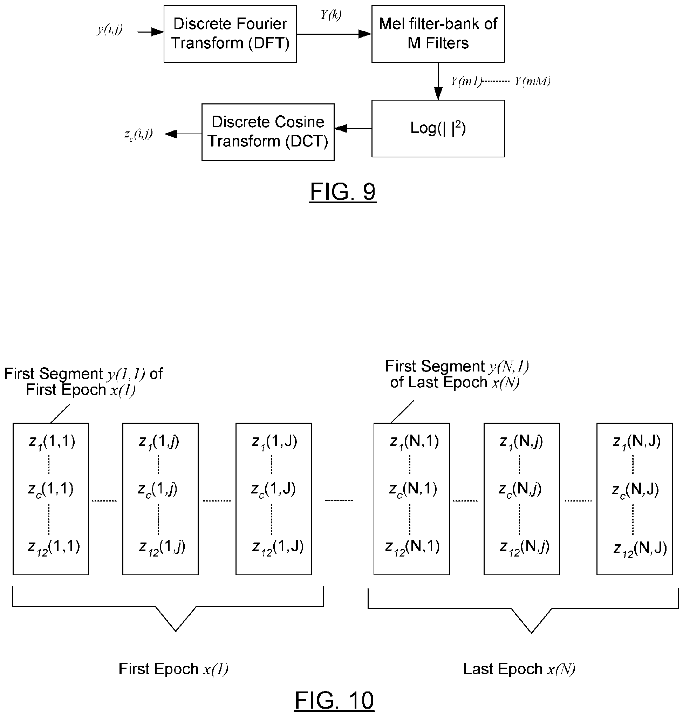

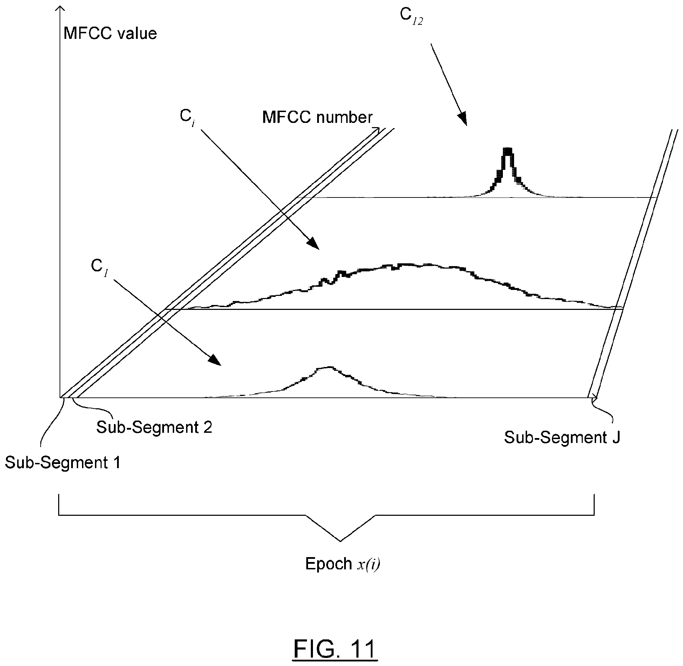

6. At box 51 (Figure 5) of the method, the Microprocessor 3 computes twelve Mel-Frequency Cepstral Coefficients (MFCC) for each of the *j* = 1,2,...,J data sub-segments y(*i,j*) as illustrated in Figure 9. It will be realized that in other embodiments of the invention more or fewer than 12 MFCCs may be calculated and that the number of MFCCs does not have to be exactly 12 for the method to work. MFCCs are modeled on the basis of the human perception of speech via the anatomical auditory system. They provide some resilience to the non-linguistic sources of variance in speech signal. The computation of MFCCs for each sub-segment y(*i,j*) is illustrated in Figure 9 in block diagram form and is well known in the prior art. Computing the MFCCs involves the estimation of short-term power spectra for each of a number of filters of a mel-frequency scale filter bank. That is done by applying a Discrete Fourier Transform 70 to the pre-emphasized sub-segments, then applying a log function 74 to the output from each of the filters of the mel-filter bank 72 and then applying a Discrete Cosine Transform (DCT) 76 to produce the MFCCs. Since the log power spectrum is real and symmetric an Inverse DFT reduces to the Discrete Cosine Transform (DCT) 76. The output from the DCT 76 comprises spectral mel-frequency cepstral coefficients (MFCCs) $z_c(i,j)$ being the $c^{th}$ coefficient of the $j^{th}$ sub-segment of the *i*th pre-emphasized epoch. Figure 10 graphically represents the MFCCs 1 to 12 vertically for each segment *j*=1,...,J of each epoch *i* = 1,...,N. Figure 11 shows a plot of three of the MFFCs $c_1$, $c_i$ and $C_{12}$ for each sub-segment across a single pre-emphasized epoch y(*i*). These plots are only for illustrative purposes to assist in understanding the procedure that is being applied by the microprocessor 3 to the digitized sound wave. It will be realized that the actual plots of MFCCs that are determined may have substantially different appearances.

7. At box 53 of Figure 5, microprocessor 3 then calculates a measure or "score" of deviation from a known statistical distribution. A number of distributions that may be used are described at the end of this specification. In the present example the statistical distribution that is used is a Gaussian distribution and the measure of deviation from Gaussian distribution is a non-Gaussianity score (NGS). The $NGS\xi_c(i)$ for each of the MFCC components over each epoch is determined. $\xi_c(i)$ represents the NGS of the $c^{th}$ MFCC component in the $i^{th}$ pre-emphasized epoch. For example, the value of $3^{rd}$ MFCC over the sub-segments in a given epoch may be close to a Gaussian distribution, in which case the NGS for the $3^{rd}$ MFCC will be a low value for that epoch, or it may be very unlike a Gaussian distribution, in which case the NGS for the $3^{rd}$ MFCC will be a high value for that epoch. The following description will mainly refer to the use of a Gaussian distribution however other statistical distributions may also be used and as previously mentioned, these are described toward the end of this specification.

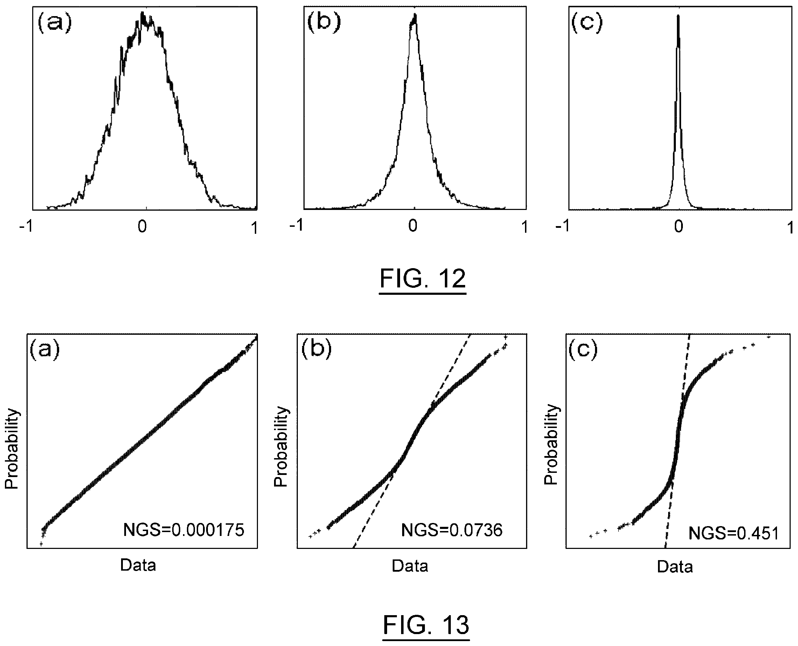

Three exemplary sample distributions are shown in Figure 12 parts (a), (b) and (c). It will be visually observed that the plot of part (a) appears to match a Gaussian "bell curve" whereas that of part (c) does not and that of part (b) is intermediate. The normal probability plot is a plot of the midpoint probability positions of a given data segment versus the theoretical quantiles of a normal distribution. If the distribution of the data under consideration is normal, the plot will be linear. Other probability distributions will lead to plots that deviate from linearity, with the particular nature and amount of deviation depending on the actual distribution itself. Figure 13 parts (a), (b), (c) show a normal (Gaussian) distribution as a linear dashed line on which corresponding plots from each of Figures 11 (a), (b) and (c) have been superimposed showing the increasing deviation from Gaussianity.

The increase in the NGS score is therefore a quantitative measure of the deviation from Gaussianity, of MFCC component values in an epoch. As previously mentioned methods of NGS computation are known in the prior art and are centered on computing the normal probability plot for each of the MFCCs for each epoch. Detailed methods of NGS computation can be found in H. Ghaemmaghami, U. Abeyratne, and C. Hukins, "Normal probability testing of snore signals for diagnosis of obstructive sleep apnea," in Engineering in Medicine and Biology Society, 2009. EMBC 2009. Annual International Conference of the IEEE, 2009, pp. 5551-5554. Figure 13 graphically represents NGS scores, normalized on a vertical scale of zero to one for each of three MFCCs (C1, Ci and C12) for each epoch 1,...,N.

At box 55 the microprocessor 3 implements a comparator and compares each NGS $\xi_c(i)$ that was computed in box 53 against a threshold $\eta$ to define $L_c(i)$ using (3).

$$L_c(i) = \begin{cases} 1 \ if \ \xi_c(i) > \eta \\ 0 \ if \ \xi_c(i) \le \eta \end{cases} \qquad (3)$$

10. At box 61 microprocessor 3 computes an MFCC-Index vector, $\Psi_c$ for all MFCC components using (4).

$$\Psi_c = \frac{\sum_i^N L_c(i)}{N}$$

11. At box 63 microprocessor 3 produces a reduced dimension test vector $\Psi_{sc\text{-}test}$ by removing some components from $\Psi_c$. As will be described, the components of $\Psi_c$ that are removed have been previously judged to have little, or no, influence on the diagnosis.

12. At box 65 the test vector is applied to the pre-trained LRM 20. It will be realised that other types of decision machines or "classifiers" can be used as well. The output of the LRM is a signal that represents a number that is "1" or very close thereto and so indicates a diagnosis of "OSA present" or "0" or very close thereto and so indicates a diagnosis of "no OSA present".

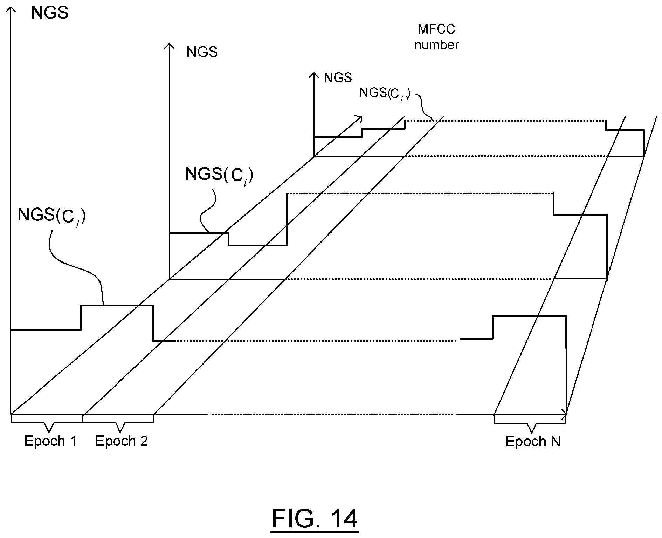

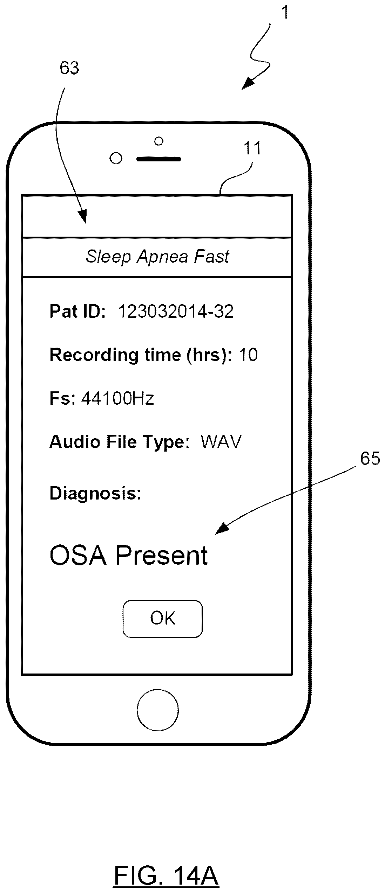

13. At box 67 the microprocessor 3 operates LCD touch screen interface 11 to present the diagnosis screen 63 in respect of patient 2 to the carer 4 as shown in Figure 14A. Diagnosis screen 63 includes a message 65 indicating the diagnosis of OSA in the particular patient 2.

*PRODUCING THE LRM*

In order to create the trained Logistic Regression Machine (LRM) 20 the Inventors initially recorded sounds from Q=41 patients including individuals with symptoms such as daytime sleepiness, snoring, tiredness lethargy etc. and who were suspected of OSA. It will be realised that a similar procedure is followed in order to train the LRM for detection of other maladies and that in that case sounds would be recorded from patients suffering from the malady in question.

The steps that have previously been described in relation to boxes 43 to 61 of Figures 4 and 5 were then performed in respect of each of the Q patients in the database and a feature matrix M of the size Q × $\Psi_c$ was formed. Q represents the total number of patients and $\Psi_c$ represents a feature vector from each patient.

*Pattern Classifier*

As previously discussed, App 6 includes instructions for implementation of a logistic-regression model (LRM) as the "pattern classifier" or "decision machine" for classifying test patient sounds as suffering from a malady being OSA in the exemplary embodiment. It will be realized that in other embodiments of the invention other types of decision machine may also be used such as trained neural nets, Bayesian decision machines and support vector machines and that other maladies, such as those that have previously been referred to may be the subject of the training of the pattern classifier or decision machine.

The LRM that is implemented by App 6 in the present embodiment of the invention is the best LRM that could be determined by the methodology that the Inventors have devised and which will now be described.

An LRM is a generalized linear model, which uses several independent features to estimate the probability of a categorical event (dependent variable). In the present case, the dependent variable Y is assumed to be equal to 'one' (Y=1) for 'OSA' subjects and 'zero' for 'non-OSA subjects. OSA and non-OSA subjects were defined using 3 different AHI thresholds, AHI = [5; 15; 30;]. These AHI thresholds are routinely used in the clinical practice to define the severity of OSA as follows:

$$
\begin{array}{ll}
\text{AHI} < 5 & \text{no OSA} \\
5 \leq \text{AHI} < 15 & \text{mild OSA} \\
15 \leq \text{AHI} < 30 & \text{moderate OSA} \\
\text{AHI} \geq 30 & \text{severe OSA}
\end{array}
$$

As is known in the prior art, an LRM model is derived using a regression function to estimate the probability Y given the independent features in $\Psi_c$ as follows:

$$Prob\left(Y = 1|_{\Psi_1, \Psi_2, ..., \Psi_c}\right) = \frac{e^w}{e^w + 1} \qquad (5)$$

$$w = \beta_0 + \beta_1 \cdot \Psi_1 + \beta_2 \cdot \Psi_2 + \cdots + + \beta_c \cdot \Psi_c \qquad (6)$$

In (6), $\beta_0$, is called the intercept and $\beta_1$, $\beta_2$ and so on are called the regression coefficients of independent variables. To select the optimal decision threshold $\lambda$ from Y (that subject is OSA if Y> $\lambda$; non-OSA otherwise) the Receiver-Operating Curve (ROC) analysis was used.

The Inventors used a K-fold cross validation (KCV) technique for the LRM design, setting K=10. In KCV technique, subject population in the database is randomly partitioned into K-equal size non-overlapping subsamples. Then of the K subsamples, data from subjects in K-1 subsamples are used to train the LRM model and data from subjects in the remaining one subsample is used to test the model. This process is systematically repeated K times such that each patient in the database is used to test the model exactly one time. At the end of this process, we end up with K different LRM models. To evaluate the performance of the designed K LRMS, performance measures such as Sensitivity (Sn), Specificity (Sp), Accuracy (Ac), Positive Predicted Value (PPV) and Negative Predicted Value (NPV) were computed.

*Feature Selection*

Feature selection is a technique of selecting a subset of features for building a robust classifier. Optimal feature selection requires the exhaustive search of all possible subsets of features. However, it is impractical to do so when large numbers of features are used as candidate features. Therefore, an alternative approach was used based on *p*-value to determine significant features. During LRM design, a *p*-value can be computed for each feature to indicate how significant that feature is to the model. Important features have low *p*-value. The Inventors used this property of an LRM to select a reasonable combination of features that facilitate the classification, in the model during the training phase. The technique that was used consisted of computing the mean *p*-value associated with $\Psi_c$ for K LRM models. Then selecting the features with mean *p*-value less than a threshold $p_{ths}$. Let $\Psi_{sc}$ be the feature vector with subset of the selected MFCC component index and $M_{fs}$ (of size Q $\times \Psi_{sc}$) be the feature matrix computed from selected features.

Once the significant features were known and selected they were used to build a new set of LRMs, following K-fold cross validation (K=10) as previously described. At the end of this process, $K_{fs}$ number of LRMs were produced using the selected features.

As previously mentioned, the Inventors used breathing sound data from Q=41 subjects. According to AHI severity these subjects were divided into four groups namely:

(i) Group 1, non-OSA subjects with RDI<5
(ii) Group 2, $5 \leq$ AHI < 15, mild OSA,

(iii) Group 3, 15 ≤ AHI < 30, moderate OSA and

(iv) Group 4, AHI ≥ 30, Severe OSA.

Table 1 sets out the demographic details of the subjects in the database for four subject groups.

Table 1: Demographic details of the subjects.

|  | Group 1 Non-OSA (RDI<5) | Group 2 Mild OSA (5≤RDI<15) | Group 3 Moderate OSA (15≤RDI<30) | Group 4 Sever OSA (RDI≥30) |
|---|---|---|---|---|
| N | 7 | 16 | 5 | 13 |
| Age | 50±14 | 53±12 | 63±10 | 56±12 |
| M:F | 4:3 | 9:7 | 5:0 | 12:1 |
| BMI | 27±3 | 36±10 | 30±3.7 | 40±8 |
| AHI | 1.76±1.2 | 8.68±2.5 | 23.08±3 | 62.58±24 |

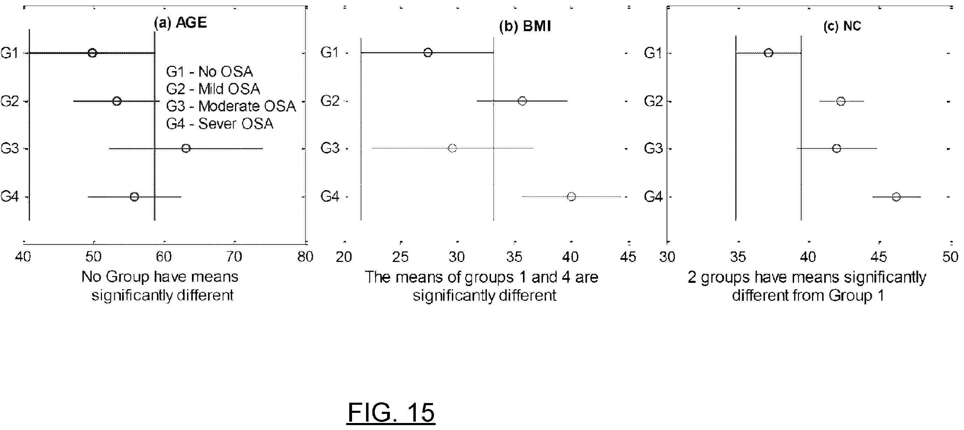

Figure 15 shows plots for mean (a) Age; (b) Body Mass Index (BMI) and (c) Neck Circumference (NC) with 95% confidence interval for subject groups. One way analysis of variance (ANOVA) statistical test showed no significant difference between the mean Age of subjects among the groups. Quite interestingly mean BMI of the severe OSA subjects was significantly higher than for non-OSA subjects. Similarly mean NC of the non-OSA subjects is significantly lower than mean NC of mild OSA and severe OSA.

*Comparison between different file formats*

One of the Inventors' objectives was to evaluate the effect of data compression on the classifier performance. For this the nocturnal breathing sound audio data was recorded from subjects in raw audio data format, WAV format. Then using *Adobe Audition*™ the data was converted into FLAC (loss-less audio format) and Mp3 (lossy audio data format).
The average length of the audio data recordings from Q = 41 subjects were 7 hours and 4 minutes with standard deviation of 1 hour and 38 minutes. The average size of an audio data recording with Fs = 44100 Hz, were, WAV file = 2.25±0.24 Giga bytes, FLAC file = 0.95±0.11 Giga bytes and that of MP3 file = 0.61±0.06 Giga bytes. On average size of a FLAC audio data file with Fs = 44100 Hz was 58±5% smaller than that of WAV file and Mp3 audio data file was 73±0.04% smaller than that of WAV file.
The Inventors investigated a snore sound waveform and its spectrogram using different audio file formats and at different sampling rates. They found no difference between the WAV file format and the FLAC file format and no difference in the time domain or in the frequency domain at all the sampling rates. With respect to the Mp3 audio file, no obvious changes could be seen in the time domain signal however a clear attenuation of the higher frequencies could be seen in the spectrogram. However high frequency attenuation could only be seen at Fs = 44100 Hz and was not present at Fs = 8000 Hz or 2000 Hz.

*Classification results - comparison between file format at Fs = 44100*

As previously discussed the LRM were trained using $\Psi_c$ feature vectors which were derived from MFCC and NGS following a K-fold cross validation technique to classify patients into OSA and non-OSA. The LRM were trained to classify patients into OSA and non-OSA at different AHI thresholds of [5; 15; 30;]. The LRM were initially trained using all features and then the LRM models were retrained using a selected sub-set of features.
Table 2 gives the test classification results for OSA diagnosis at different AHI thresholds optimized for epoch lengths. These results are for audio data sampled at Fs = 44,100 Hz.

Table 2: Cross validation results for OSA and non-OSA classification at different AHI thresholds.

| AHI = 5 | | | | | | | |
|---|---|---|---|---|---|---|---|
|  | EPL | Sensitivity | Specificity | PPV | NPV | Accuracy | Features |
| WAV | 10 | 85 | 43 | 88 | 38 | 78 | All |
|  | 10 | 94 | 86 | 97 | 75 | 93 | [8,10,11,12] |

(continued)

| AHI = 5 | | | | | | | |
|---|---|---|---|---|---|---|---|
| | EPL | Sensitivity | Specificity | PPV | NPV | Accuracy | Features |
| FLAC | 10 | 85 | 43 | 88 | 38 | 78 | All |
| | 10 | 94 | 86 | 97 | 75 | 93 | [8,10,11,12] |
| MP3 | 30 | 88 | 29 | 86 | 33 | 78 | All |
| | 30 | 88 | 86 | 97 | 60 | 88 | [2,5,8,10] |

| AHI = 15 | | | | | | | |
|---|---|---|---|---|---|---|---|
| | EPL | Sensitivity | Specificity | PPV | NPV | Accuracy | Features |
| WAV | 30 | 61 | 78 | 69 | 72 | 71 | All |
| | 30 | 83 | 91 | 88 | 88 | 88 | [3,9] |
| FLAC | 30 | 61 | 78 | 69 | 72 | 71 | All |
| | 30 | 83 | 91 | 88 | 88 | 88 | [3,9] |
| MP3 | 20 | 72 | 78 | 72 | 78 | 76 | All |
| | 20 | 83 | 87 | 83 | 87 | 85 | [1,2,3,7,9] |

| AHI = 30 | | | | | | | |
|---|---|---|---|---|---|---|---|
| | EPL | Sensitivity | Specificity | PPV | NPV | Accuracy | Features |
| WAY | 10 | 100 | 75 | 65 | 100 | 83 | All |
| | 10 | 100 | 93 | 87 | 100 | 95 | [1,2,3,6,10,11] |
| FLAC | 10 | 100 | 75 | 65 | 100 | 83 | All |
| | 10 | 100 | 93 | 87 | 100 | 95 | [1,2,3,6,10,11] |
| MP3 | 30 | 92 | 79 | 67 | 96 | 83 | All |
| | 30 | 92 | 89 | 80 | 96 | 90 | 3 |

It will be observed from Table 2 that there is no difference in classification accuracy between WAV and FLAC audio data at all the AHI thresholds. When selected features are used for model training, WAV and FLAC audio format have classification sensitivities/specificities of 94/86%, 83/91% and 100/93% respectively at AHI = 5, 15 and 30. Classification results using Mp3 audio data format were slightly lower than WAV/FLAC audio data format. The sensitivities/specificities of the Mp3 data was 88/86%, 83/87 and 92/89% respectively at AHI = 5, 15 and 30.

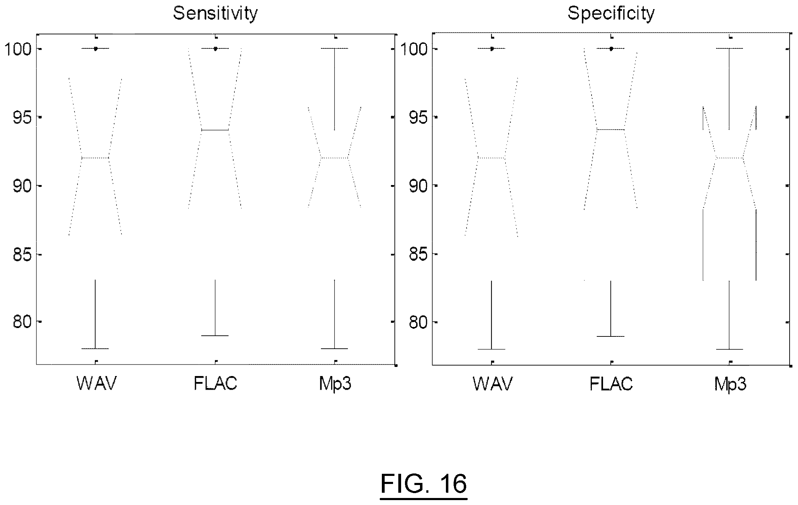

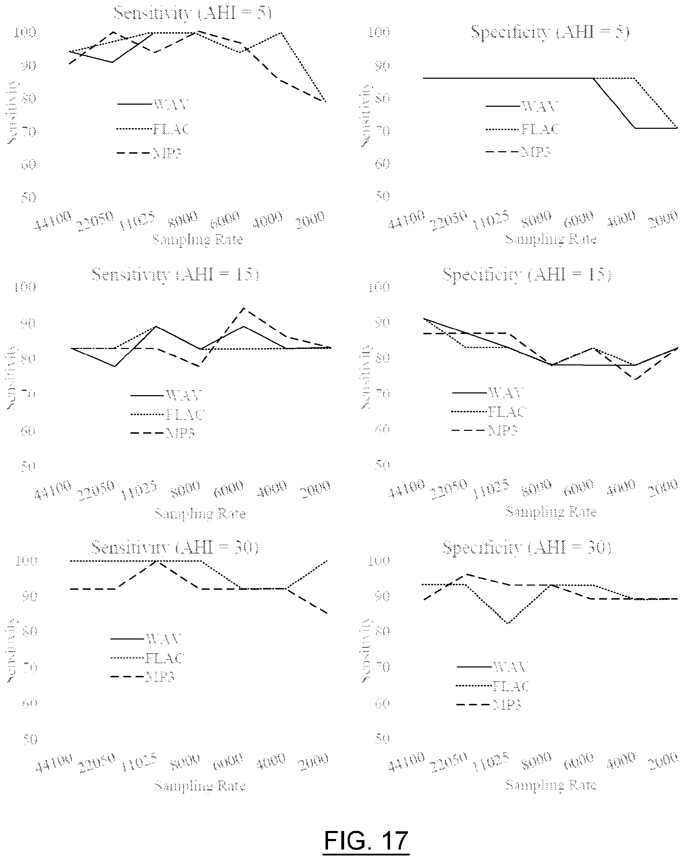

Figure 16 shows the boxplot of test classification Sensitivity and Specificity for the three types of audio data file format. To generate this graph, results of LRM from a file format, at different AHI thresholds and at different Fs were pooled together. According to Fig. 17, the LRM show no significance (p = 0.47) difference in classification performance when trained using MFCC features computed with different file format.

*Classification result - effect of sampling frequency*

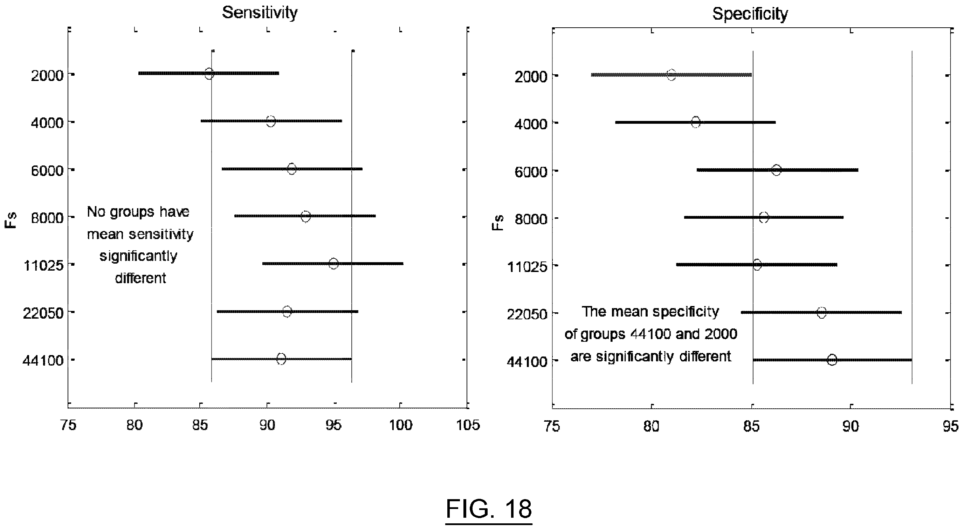

As previously discussed, the patient sounds may be resampled with different sampling frequencies Fs = [22050; 11025; 8000; 6000; 4000; 2000; 1000; 500; 200;] Hz. Note that audio data is initially recorded at Fs = 44100 Hz. MFCC features were then computed with resampled data. Figure 17 illustrates the variation in model test classification performance (sensitivity and specificity) with different Fs. Results in Fig. 17 are from selected MFCC features. Figure 18 shows the mean test classification Sensitivity and Specificity with 95% confidence interval, achieved for audio data with different sampling rate Fs. To generate the graph in Fig. 18, results at specific Fs from three file formats at different AHI thresholds were pooled together. According to Fig.17 and Fig.18 a gross variation in sensitivity and specificity with change in data sampling rate can be seen. In general, Sensitivity initially increases with decrease in Fs, reaching at its peak at Fs = 11025 Hz. It then starts decreasing, reaching its lowest value at Fs = 2000 Hz across all file formats. Note that though a decrease in Sensitivity can be seen at lower Fs, however this decrease is insignificant. In Specificity generally remained stable when Fs decreased from 44100 to 22050, then it decreased at Fs = 11025 Hz. Then from Fs = 11025 Hz it starts increasing up-to Fs = 6000 Hz and then gain starts decreasing reaching lowest at Fs = 2000 Hz. The decrease in Specificity was significant but only with respect to Fs = 44100.

The results indicate that methods according to embodiments of the present invention can classify patients into OSA and non-OSA at different AHI threshold with a high accuracy.

In the past several researchers [10-16] have attempted to use snoring sounds to diagnose OSA and many of the existing methods [10-12, 14] have depended on the identification of snore segments from the overnight sound data. Hence if the snore segmentation algorithm fails to identify any snore segments or if the patient did not snore then results of the test will be indeterminate.

In contrast to those previous methods that have relied on detection of snore segments in the patient sound for subsequent diagnosis of OSA, preferred embodiments of the invention described herein capture the instantaneous characteristics of the upper airway present in continuous recordings of the breath sound.

Furthermore, preferred embodiments of the invention make use of MFCC features for the diagnosis of OSA via measuring the amount of deviation of MFCC features from Gaussianity in a given sound segment ("epoch"). This approach has the advantage of better performance, robustness against AHI variation and low computational complexity as it does not depend on identifying snore segments from breath sound data.

The Inventors' results also illustrate that it is possible to record the patient sounds, i.e. the sounds of the patient breathing, with a compressed audio format and at a low sampling rate without compromising on classification accuracies. The results show that it is possible to achieve a sensitivity/specificity of 97/86 %, 94/83 % and 92/89 % respectively at AHI threshold of 5, 15 and 30, with breath sound data recorded using Mp3 file format at Fs = 6000 Hz (Fig. 17). With these settings the memory space required to record breath sound data of 8 hours duration will be < 28 megabytes which is important where it is desired to store lengthy sounds from a number of patients. The automated, non-contact and mobile technology according to preferred embodiments of the present invention provides an excellent tool for population screening.

Previously in Figure 1 a block diagram of an OSA diagnostic device 1 according to a preferred embodiment of the present invention was provided and discussed. It is also possible in another embodiment of the invention to provide a dedicated OSA diagnostic device rather one that is comprised of a specially programmed microprocessor based apparatus such as a specially programmed smartphone.

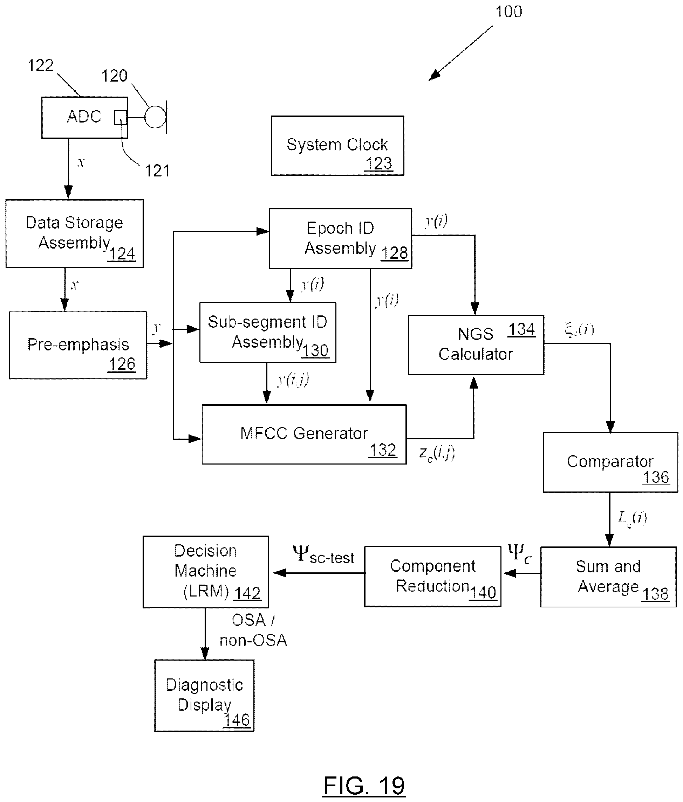

A dedicated OSA diagnostic apparatus 100 for diagnosing the presence of Obstructive Sleep Apnea (OSA) of a patient 2 is illustrated in Figure 19. The apparatus includes a system clock 123 for synchronizing the various modules of the apparatus. A microphone 120 is coupled via an anti-aliasing filter 121 to an analog-to-digital converter (ADC) 122. The output from the ADC 122 is received by an electronic storage assembly 124, which stores a digitized audio file of patient sounds from the audio interface ADC 122. A pre-emphasis assembly 126 is coupled to the output of the data storage assembly 124 for applying pre-emphasis to the digitized audio signal.

The apparatus 100 includes an epoch identification assembly 128 that is coupled to an output side of the pre-emphasis assembly 126 to process the digitized audio file and identify a number of epochs in the audio file. A sub-segment identification assembly 130 is provided that is arranged to process the digitized audio file and identify a plurality of sub-segments therein for each of the epochs.

The sub-segment ID assembly 130 and the Epoch ID Assembly 128 provide respective outputs to the Mel-Frequency Cepstral Coefficient generator 132 which processes the digitized audio file from the pre-emphasis assembly 126 to produce a multiplicity of mel-frequency cepstral coefficients (MFCCs) signals for each of the sub-segments.

A non-Gaussianity Score calculation assembly 134 is provided that is responsive to the Mel-Frequency Cepstral Coefficient generator and which is arranged to process the MFCC signals from the MFCC generator 132 for each of the sub-segments to produce NGS scores for each of the MFCCs signals for each epoch as identified by the Epoch ID Assembly 128. In other embodiments of the invention a deviation from probability distribution score calculation assembly may be used to calculate a score for deviation from another distribution other than Gaussian.

The output from the NGS calculator 134 is passed to a comparator 136 which compares each of the MFCCs to a threshold value and respectively outputs a "0" or a "1" if the MFCC value is below or above threshold.

The output from the comparator is summed and averaged by Sum-and-Average block 138 to produce an initial test-vector which is subsequently reduced in dimension by Component Reduction assembly 140 to produce a reduced MFCC feature test vector. The reduced MFCC feature test vector is then passed to a decision machine block 142 which generates an OSA / non-OSA signal in response to the reduced MFCC feature test vector.

The apparatus 100 includes a human-machine interface including diagnostic display 146 that is coupled to the decision machine block 142 and which is arranged to present the OSA diagnosis to a human.

Whilst the previous discussion focused on a method and apparatus according to a preferred embodiment of the invention that uses deviation from Gaussian distribution, other measures of deviation from a known statistical distribution may also be used in other embodiments of the present invention and some of these are listed below. In other embodiments App 6 may include instructions for microprocessor 5 to implement each of the following statistical techniques as an alternative to determining deviation from Gaussian distribution.

1. Compute the Chi-squared test statistic between the MFCC distribution and the target distribution (e.g. Gaussian) and use it directly as a feature to feed the classifier.

2. Compute p-values for the Chi-squared test statistic between the MFCC distribution and the target distribution (e.g. Gaussian) and use the p-value directly as a feature to feed the classifier.

3. Use the KS test (Kolmogorov-Smirnov) test statistic in the place of Chi-squared described in (1) and (2) above

4. Use the Lilliefors test for normalcy as described in (1) and (2) above, with the Gaussian distribution.

5. Use a combination of deviation measures, including the NGS measure.

Results on the above methods 1-5 are set forth below.

**Non-segmentation based OSA Classification results (Scored using 2007 Alternate Criteria):**

**Data statistics for 73 usable recordings:**

|  |  |  |
|---|---|---|
| RDI<15 | = | 39 |
| RDI>=15 | = | 34 |
| RDI=>30 | = | 22 |
| Male | = | 42 |
| Female | = | 31 |

**Training and Leave-One-Out Validation results:**

**Results Summary (RDI threshold = 15):**

| EPOCH LENGTH = 30 | Training Results | | Leave One Out Validation Results | |
|---|---|---|---|---|
|  | Sensitivity | Specificity | Sensitivity | Specificity |
| **NGS of MFCC** | 91.26 | 92.38 | 91.18 | 92.31 |
| **Chi Sq Test of Mfcc (T-stat value)** | 88.52 | 89.99 | 88.24 | 84.62 |
| **KS test of MFCC (T-stat value)** | 88.07 | 89.60 | 88.24 | 84.62 |
| **Lilliefors test of MFCC (T-stat value)** | 91.17 | 92.31 | 91.18 | 87.18 |
| **Chi Sq Test of Mfcc (p- value)** | 87.91 | 89.46 | 85.29 | 84.62 |
| **KS test of MFCC (p- value)** | 88.07 | 89.60 | 88.24 | 87.18 |
| **Lilliefors test of MFCC (p-value)** | 88.44 | 89.92 | 85.29 | 84.62 |
| **NGS + Lilliefors test** | 95.65 | 97.40 | 91.18 | 97.44 |
| EPOCH LENGTH = 15 | Training Results | | Leave One Out Validation Results | |
|  | Sensitivity | Specificity | Sensitivity | Specificity |
| **NGS of MFCC** | 91.13 | 92.27 | 91.18 | 92.31 |
| **Chi Sq Test of Mfcc (T-stat value)** | 85.05 | 86.96 | 85.29 | 84.62 |
| **KS test of MFCC (T-stat value)** | 90.97 | 92.13 | 85.29 | 87.18 |
| **Lilliefors test of MFCC (T-stat value)** | 93.95 | 94.73 | 88.24 | 87.18 |
| **Chi Sq Test of Mfcc (p-value)** | 84.60 | 86.57 | 85.29 | 84.62 |

(continued)

| EPOCH LENGTH = 15 | Training Results | | Leave One Out Validation Results | |
|---|---|---|---|---|
| | Sensitivity | Specificity | Sensitivity | Specificity |
| **KS test of MFCC (p- value)** | 88.07 | 89.60 | 88.24 | 87.18 |
| **Lilliefors test of MFCC (p-value)** | 85.09 | 87.00 | 85.29 | 84.62 |
| **NGS + Lilliefors test** | 91.13 | 92.27 | 91.18 | 92.31 |

## Dividing Data into Training and Testing & objectively removing Noisy recordings

| | Training Set | Test Set |
|---|---|---|
| **Number of Subjects** | 53 | 20 |
| **Male** | 31 | 11 |
| **Female** | 22 | 9 |
| **RDI<15** | 29 | 10 |
| **RDI≥15** | 24 | 10 |

## SET 1 [Train and LOV = 53 and Independent Test = 20]:

| EPOCH LENGTH = 15 | Training Results | | Leave One Out Validation Results | | Test Results | |
|---|---|---|---|---|---|---|
| | Sensitivit y | Specificity | Sensitivity | Specificity | Sensitivit y | Specif icity |
| **NGS of MFCC** | 87.99 | 89.29 | 88.00 | 89.29 | 70 | 100 |
| **Chi Sq Test of Mfcc (T-stat value)** | 87.92 | 89.21 | 88.00 | 82.14 | 70 | 60 |
| **KS test of MFCC (T-stat value)** | 91.84 | 92.72 | 92.00 | 85.71 | 50 | 80 |
| **Lilliefors test of MFCC (T-stat value)** | 85.76 | 87.29 | 88.00 | 85.71 | 80 | 90 |
| **Chi Sq Test of Mfcc (p- value)** | 83.99 | 85.71 | 84.00 | 82.14 | 70 | 40 |
| **KS test of MFCC (p-value)** | 87.76 | 89.08 | 88.00 | 85.71 | 90 | 80 |
| **Lilliefors test of MFCC (p-value)** | 87.99 | 89.28 | 84.00 | 89.29 | 80 | 80 |
| **NGS + Lilliefors test p value** | 91.06 | 92.03 | 92 | 89.29 | 70 | 100 |

| EPOCH LENGTH = 30 | Training Results | | Leave One Out Validation Results | | Test Results | |
|---|---|---|---|---|---|---|
| | Sensiti vity | Specific ity | Sensitivity | Specificity | Sensitivity | Specificity |
| NGS of MFCC | 95.53 | 96.02 | 92.00 | 89.29 | 80 | 70 |
| Chi Sq Test of Mfcc (T-stat value) | 87.76 | 89.08 | 88.00 | 85.71 | 80 | 70 |
| KS test of MFCC (T-stat value) | 91.92 | 92.78 | 92.00 | 85.71 | 50 | 70 |
| Lilliefors test of MFCC (T-stat value) | 88.38 | 89.63 | 88.00 | 89.29 | 80 | 80 |
| Chi Sq Test of Mfcc (p- value) | 91.76 | 92.65 | 88.00 | 89.29 | 60 | 70 |
| KS test of MFCC (p-value) | 87.53 | 88.87 | 88.00 | 85.71 | 60 | 90 |
| Lilliefors test of MFCC (p- value) | 88.07 | 89.35 | 88.00 | 85.71 | 80 | 70 |
| NGS + Lilliefors test p value | 95.84 | 96.29 | 96 | 92.86 | 80 | 90 |

**SET 2 [Created after shuffling the training and test data; Train and LOV = 53 and Independent Test = 20] :**

| EPOCH LENGTH = 15 | Training Results | | Leave One Out Validation Results | | Test Results | |
|---|---|---|---|---|---|---|
| | Sensitivity | Specificity | Sensitivity | Specificity | Sensiti vity | Specific ity |
| NGS of MFCC | 92.08 | 92.92 | 92.00 | 89.29 | 90 | 80 |
| Chi Sq Test of Mfcc (T-stat value) | 87.92 | 89.22 | 88.00 | 89.29 | 60 | 70 |
| KS test of MFCC (T-stat value) | 88.15 | 89.42 | 88.00 | 85.71 | 80 | 50 |
| Lilliefors test of MFCC (T-stat value) | 88.07 | 89.35 | 88.00 | 89.29 | 60 | 60 |
| Chi Sq Test of Mfcc (p- value) | 87.84 | 89.15 | 88.00 | 89.29 | 80 | 60 |
| KS test of MFCC (p-value) | 88.38 | 89.63 | 88.00 | 85.71 | 80 | 80 |
| Lilliefors test of MFCC (p- value) | 88.00 | 89.28 | 88.00 | 85.71 | 80 | 80 |
| NGS + Lilliefors test p value | 91.76 | 92.65 | 92 | 89.28 | 80 | 60 |

(continued)

| EPOCH LENGTH = 30 | Training Results | | Leave One Out Validation Results | | Test Results | |
|---|---|---|---|---|---|---|
| | Sensitivity | Specificity | Sensitivity | Specificity | Sensiti vity | Specificit y |
| NGS of MFCC | 91.76 | 92.65 | 92.00 | 89.29 | 80 | 90 |
| Chi Sq Test of Mfcc (T-stat value) | 87.76 | 89.08 | 88.00 | 85.71 | 80 | 70 |
| KS test of MFCC (T-stat value) | 87.92 | 89.22 | 88.00 | 89.29 | 80 | 80 |
| Lilliefors test of MFCC (T-stat value) | 91.69 | 92.58 | 92.00 | 89.29 | 90 | 80 |
| Chi Sq Test of Mfcc (p- value) | 87.99 | 89.29 | 88.00 | 89.29 | 70 | 60 |
| KS test of MFCC (p-value) | 88.46 | 89.69 | 88.00 | 89.29 | 80 | 80 |
| Lilliefors test of MFCC (p- value) | 87.99 | 89.29 | 88.00 | 89.29 | 50 | 80 |
| NGS + Lilliefors test p value | 100 | 100 | 96 | 92.86 | 90 | 60 |

**SET 3 [Created after shuffling the training and test data; Train and LOV = 53 and Independent Test = 20]:**

| EPOCH LENGTH = 15 | Training Results | | Leave One Out Validation Results | | Test Results | |
|---|---|---|---|---|---|---|
| | Sensitivity | Specificity | Sensitivity | Specificity | Sensiti vity | Specific ity |
| NGS of MFCC | 91.84 | 92.72 | 92.00 | 89.29 | 80 | 80 |
| Chi Sq Test of Mfcc (T-stat value) | 87.84 | 89.14 | 88.00 | 85.71 | 80 | 70 |
| KS test of MFCC (T-stat value) | 87.99 | 89.29 | 88.00 | 89.29 | 70 | 70 |
| Lilliefors test of MFCC (T-stat value) | 91.84 | 92.72 | 92.00 | 89.29 | 80 | 70 |
| Chi Sq Test of Mfcc (p- value) | 87.84 | 89.14 | 88.00 | 85.71 | 60 | 60 |
| KS test of MFCC (p-value) | 92.00 | 92.85 | 92.00 | 85.71 | 30 | 60 |
| Lilliefors test of MFCC (p- value) | 92.00 | 92.85 | 92.00 | 89.29 | 80 | 70 |
| NGS + Lilliefors test p value | 92 | 92.85 | 92 | 89.28 | 90 | 80 |

(continued)

| EPOCH LENGTH = 30 | Training Results | | Leave One Out Validation Results | | Test Results | |
|---|---|---|---|---|---|---|
| | Sensitivity | Specificity | Sensitivity | Specificity | Sensiti vity | Specific ity |
| **NGS of MFCC** | 91.84 | 92.72 | 92.00 | 89.29 | 90 | 80 |
| **Chi Sq Test of Mfcc (T-stat value)** | 92.00 | 92.85 | 92.00 | 89.29 | 60 | 60 |
| **KS test of MFCC (T-stat value)** | 91.76 | 92.65 | 92.00 | 89.29 | 90 | 60 |
| **Lilliefors test of MFCC (T-stat value)** | 88.31 | 89.56 | 88.00 | 89.29 | 70 | 60 |
| **Chi Sq Test of Mfcc (p- value)** | 91.46 | 92.37 | 88.00 | 89.29 | 50 | 70 |
| **KS test of MFCC (p-value)** | 91.69 | 92.58 | 88.00 | 89.29 | 40 | 50 |
| **Lilliefors test of MFCC (p- value)** | 87.99 | 89.29 | 88.00 | 89.29 | 80 | 80 |
| **NGS + Lilliefors test p value** | 91.84 | 92.72 | 92 | 89.29 | 90 | 80 |

*Iphone* Data Analysis:

Total *Iphone* dataset available = 83

Scored with 2007 Alternate = 81 [data recorded between 2010 and 2014]

Scored with 2012 Recommended = 2 [data recorded from 2015 onward]

Non-Segmentation based analysis (Scored using 2007

Alternate criteria):

Total dataset available = 81

RDI Missing = 4

CPAP study = 2

Audio recording corrupt = 3 (4/11/2014; 7/11/2014P1; 24/1/2013P2)

Objective Noise detection algorithm rejected 2 recordings based on excessive noise = 2 (19/11/2012P1; 31/01/2013P1)

**Data statistics for 70 usable recordings:**

```
RDI<15       = 38
RDI>=15      = 32
RDI=>30      = 20
Male         = 40
Female       = 30
```

**Dividing Data into Training and Testing & objectively removing Noisy recordings**

Buffer Size = 8;

**SET 1 [Train and LOV = 50 and Independent Test = 20]:**

| EPOCH LENGTH = 15 | Training Results | | Leave One Out Validation Results | | Test Results | |
|---|---|---|---|---|---|---|
| | Sensitivity | Specificity | Sensitivity | Specificity | Sensitivity | Specificity |
| **NGS of MFCC** | 84.44 | 86.30 | 86.36 | 85.71 | 70 | 80 |
| **Chi Sq Test of Mfcc (T-stat value)** | 84.35 | 86.16 | 86.36 | 85.71 | 70 | 70 |
| **KS test of MFCC (T-stat value)** | 83.99 | 85.86 | 86.36 | 85.71 | 60 | 80 |
| **Lilliefors test of MFCC (T-stat value)** | 91.74 | 93.51 | 86.36 | 89.29 | 60 | 80 |
| **Chi Sq Test of Mfcc (p- value)** | 86.35 | 89.28 | 86.36 | 85.71 | 70 | 80 |
| **KS test of MFCC (p- value)** | 83.99 | 85.86 | 86.36 | 85.71 | 60 | 80 |
| **Lilliefors test of MFCC (p- value)** | 86.45 | 88.29 | 86.36 | 85.71 | 60 | 80 |
| **NGS + Lilliefors test p value** | 100.00 | 100.00 | 90.91 | 89.29 | 60 | 50 |

| EPOCH LENGTH = 30 | Training Results | | Leave One Out Validation Results | | Test Results | |
|---|---|---|---|---|---|---|
| | Sensitivity | Specificity | Sensitivity | Specificity | Sensitivity | Specificity |
| **NGS of MFCC** | 91.00 | 92.93 | 86.36 | 89.29 | 70 | 80 |
| **Chi Sq Test of Mfcc (T-stat value)** | 86.36 | 87.92 | 86.36 | 85.71 | 80 | 80 |
| **KS test of MFCC (T-stat value)** | 90.91 | 92.85 | 90.91 | 85.71 | 60 | 60 |

(continued)

| EPOCH LENGTH = 30 | Training Results | | Leave One Out Validation Results | | Test Results | |
|---|---|---|---|---|---|---|
| | Sensitivity | Specificity | Sensitivity | Specificity | Sensitivity | Specificity |
| Lilliefors test of MFCC (T-stat value) | 92.02 | 93.73 | 86.36 | 89.29 | 70 | 80 |
| Chi Sq Test of Mfcc (p- value) | 90.81 | 92.78 | 90.91 | 89.29 | 80 | 80 |
| KS test of MFCC (p- value) | 90.91 | 92.85 | 90.91 | 85.71 | 60 | 60 |
| Lilliefors test of MFCC (p- value) | 86.64 | 89.50 | 86.36 | 85.71 | 60 | 80 |
| NGS + Lilliefors test p value | 100.00 | 100.00 | 95.45 | 89.29 | 80 | 50 |

**SET 2 [Created after shuffling the training and test data; Train and LOV = 50 and Independent Test = 20]:**

| EPOCH LENGTH = 15 | Training Results | | Leave One Out Validation Results | | Test Results | |
|---|---|---|---|---|---|---|
| | Sensitivity | Specificity | Sensitivity | Specificity | Sensiti vity | Specif icity |
| NGS of MFCC | 86.08 | 87.71 | 86.36 | 85.71 | 100 | 50 |
| Chi Sq Test of Mfcc (T-stat value) | 83.90 | 85.71 | 86.36 | 85.71 | 80 | 20 |
| KS test of MFCC (T-stat value) | 83.71 | 85.57 | 86.36 | 85.71 | 80 | 50 |
| Lilliefors test of MFCC (T-stat value) | 91.00 | 92.93 | 90.91 | 92.86 | 70 | 50 |
| Chi Sq Test of Mfcc (p- value) | 90.91 | 92.85 | 86.36 | 85.71 | 70 | 60 |
| KS test of MFCC (p-value) | 83.71 | 85.57 | 86.36 | 85.71 | 80 | 50 |
| Lilliefors test of MFCC (p- value) | 86.08 | 87.71 | 86.36 | 85.71 | 80 | 30 |
| NGS + Lilliefors test p value | 87.38 | 90.01 | 86.36 | 85.71 | 70 | 60 |

| EPOCH LENGTH = 30 | Training Results | | Leave One Out Validation Results | | Test Results | |
|---|---|---|---|---|---|---|
| | Sensitivity | Specificity | Sensitivity | Specificity | Sensiti vity | Specif icity |
| NGS of MFCC | 85.81 | 87.41 | 86.36 | 85.71 | 90 | 70 |

(continued)

| EPOCH LENGTH = 30 | Training Results | | Leave One Out Validation Results | | Test Results | |
|---|---|---|---|---|---|---|
| | Sensitivity | Specificity | Sensitivity | Specificity | Sensiti vity | Specif icity |
| Chi Sq Test of Mfcc (T-stat value) | 90.72 | 92.71 | 86.36 | 89.29 | 90 | 60 |
| KS test of MFCC (T-stat value) | 83.81 | 85.64 | 86.36 | 85.71 | 80 | 60 |
| Lilliefors test of MFCC (T-stat value) | 90.72 | 92.71 | 86.36 | 89.29 | 70 | 60 |
| Chi Sq Test of Mfcc (p- value) | 90.72 | 92.71 | 86.36 | 89.29 | 80 | 60 |
| KS test of MFCC (p-value) | 83.90 | 85.71 | 86.36 | 85.71 | 60 | 40 |
| Lilliefors test of MFCC (p- value) | 83.80 | 85.64 | 81.82 | 85.71 | 90 | 50 |
| NGS + Lilliefors test p value | 85.81 | 87.41 | 86.36 | 85.71 | 90 | 70 |

SET 3 [Created after shuffling the training and test data; Train and LOV = 50 and Independent Test = 20] :

| EPOCH LENGTH = 15 | Training Results | | Leave One Out Validation Results | | Test Results | |
|---|---|---|---|---|---|---|
| | Sensitivity | Specificity | Sensitivity | Specificity | Sensitivity | Specificity |
| NGS of MFCC | 90.72 | 92.71 | 90.91 | 85.71 | 80 | 70 |
| Chi Sq Test of Mfcc (T-stat value) | 90.90 | 92.86 | 90.91 | 89.29 | 80 | 40 |
| KS test of MFCC (T-stat value) | 83.99 | 85.79 | 86.36 | 85.71 | 80 | 80 |
| Lilliefors test of MFCC (T-stat value) | 90.90 | 92.85 | 90.91 | 89.29 | 80 | 60 |
| Chi Sq Test of Mfcc (p- value) | 90.81 | 92.78 | 86.36 | 89.29 | 60 | 60 |
| KS test of MFCC (p-value) | 83.99 | 85.86 | 86.36 | 85.71 | 70 | 80 |
| Lilliefors test of MFCC (p-value) | 79.53 | 82.29 | 81.82 | 82.14 | 50 | 60 |
| NGS + Lilliefors test p value | 86.17 | 89.07 | 86.36 | 89.29 | 80 | 50 |

| EPOCH LENGTH = 30 | Training Results | | Leave One Out Validation Results | | Test Results | |
|---|---|---|---|---|---|---|
| | Sensitivity | Specificity | Sensitivity | Specificity | Sensitivity | Specificity |
| NGS of MFCC | 83.90 | 85.71 | 86.36 | 85.71 | 60 | 50 |
| Chi Sq Test of Mfcc (T-stat value) | 85.90 | 87.49 | 86.36 | 85.71 | 70 | 70 |
| KS test of MFCC (T-stat value) | 86.73 | 89.57 | 86.36 | 89.29 | 60 | 80 |
| Lilliefors test of MFCC (T-stat value) | 100.00 | 100.00 | 100.00 | 89.29 | 70 | 50 |
| Chi Sq Test of Mfcc (p- value) | 86.08 | 87.63 | 86.36 | 85.71 | 40 | 60 |
| KS test of MFCC (p-value) | 86.82 | 89.65 | 86.36 | 89.29 | 60 | 80 |
| Lilliefors test of MFCC (p-value) | 84.26 | 86.08 | 86.36 | 85.71 | 70 | 70 |
| NGS + Lilliefors test p value | 100.00 | 100.00 | 86.36 | 85.71 | 60 | 60 |

*Android Trained Model tested on Iphone Dataset*

**SET 1**

| EPOCH LENGTH = 15 | Training Set Patient | | Test Set Patient | |
|---|---|---|---|---|
| | Sensitivity | Specificity | Sensitivity | Specificity |
| NGS of MFCC | **64** | 64 | 80 | 80 |
| Chi Sq Test of Mfcc (T-stat value) | 68 | 54 | 90 | 60 |
| KS test of MFCC (T-stat value) | 55 | 75 | 50 | 80 |
| Lilliefors test of MFCC (T-stat value) | 64 | 64 | 80 | 80 |
| Chi Sq Test of Mfcc (p-value) | 77 | 39 | 80 | 40 |
| KS test of MFCC (p- value) | 55 | 61 | 40 | 70 |
| Lilliefors test of MFCC (p-value) | 68 | 64 | 90 | 80 |
| NGS + Lilliefors test p value | 59 | 75 | 80 | 70 |

| EPOCH LENGTH = 30 | Training Set Patient | | Test Set Patient | |
|---|---|---|---|---|
| | Sensitivity | Specificity | Sensitivity | Specificity |
| NGS of MFCC | 59 | 64 | 90 | 70 |
| Chi Sq Test of Mfcc (T-stat value) | 59 | 64 | 80 | 70 |
| KS test of MFCC (T-stat value) | 64 | 75 | 60 | 70 |

(continued)

| EPOCH LENGTH = 30 | Training Set Patient | | Test Set Patient | |
|---|---|---|---|---|
| | Sensitivity | Specificity | Sensitivity | Specificity |
| Lilliefors test of MFCC (T-stat value) | 64 | 68 | 90 | 80 |
| Chi Sq Test of Mfcc (p-value) | 77 | 64 | 80 | 70 |
| KS test of MFCC (p- value) | 77 | 54 | 70 | 70 |
| Lilliefors test of MFCC (p-value) | 64 | 64 | 90 | 70 |
| NGS + Lilliefors test p value | 68 | 86 | 90 | 60 |

**SET 2**

| EPOCH LENGTH = 15 | Training Set Patient | | Test Set Patient | |
|---|---|---|---|---|
| | Sensitivity | Specificity | Sensitivity | Specificity |
| NGS of MFCC | **82** | 54 | 70 | 60 |
| Chi Sq Test of Mfcc (T-stat value) | 77 | 71 | 80 | 50 |
| KS test of MFCC (T-stat value) | 68 | 71 | 60 | 60 |
| Lilliefors test of MFCC (T-stat value) | 77 | 75 | 80 | 60 |
| Chi Sq Test of Mfcc (p- value) | 73 | 82 | 90 | 50 |
| KS test of MFCC (p-value) | 68 | 68 | 50 | 50 |
| Lilliefors test of MFCC (p- value) | 77 | 54 | 70 | 40 |
| NGS + Lilliefors test p value | 86 | 61 | 70 | 30 |

| EPOCH LENGTH = 30 | Training Set Patient | | Test Set Patient | |
|---|---|---|---|---|
| | Sensitivity | Specificity | Sensitivity | Specificity |
| NGS of MFCC | 77 | 68 | 60 | 60 |
| Chi Sq Test of Mfcc (T-stat value) | 68 | 64 | 80 | 50 |
| KS test of MFCC (T-stat value) | 64 | 71 | 50 | 50 |
| Lilliefors test of MFCC (T-stat value) | 73 | 61 | 70 | 50 |
| Chi Sq Test of Mfcc (p- value) | 68 | 75 | 70 | 60 |
| KS test of MFCC (p-value) | 64 | 71 | 50 | 50 |
| Lilliefors test of MFCC (p- value) | 64 | 79 | 80 | 50 |
| NGS + Lilliefors test p value | 95 | 46 | 90 | 80 |

**SET 3**

| EPOCH LENGTH = 15 | Training Set Patient | | Test Set Patient | |
|---|---|---|---|---|
| | Sensitivity | Specificity | Sensitivity | Specificity |
| NGS of MFCC | **77** | 68 | 70 | 80 |

(continued)

| EPOCH LENGTH = 15 | Training Set Patient | | Test Set Patient | |
|---|---|---|---|---|
| | Sensitivity | Specificity | Sensitivity | Specificity |
| Chi Sq Test of Mfcc (T-stat value) | 77 | 61 | 80 | 70 |
| KS test of MFCC (T-stat value) | 50 | 61 | 50 | 80 |
| Lilliefors test of MFCC (T-stat value) | 77 | 61 | 70 | 70 |
| Chi Sq Test of Mfcc (p- value) | 68 | 79 | 60 | 80 |
| KS test of MFCC (p-value) | 45 | 71 | 40 | 60 |
| Lilliefors test of MFCC (p- value) | 73 | 64 | 70 | 70 |
| NGS + Lilliefors test p value | 82 | 61 | 60 | 90 |
| | | | | |
| EPOCH LENGTH = 30 | Training Set Patient | | Test Set Patient | |
| | Sensitivity | Specificity | Sensitivity | Specificity |
| NGS of MFCC | 68 | 64 | 80 | 60 |
| Chi Sq Test of Mfcc (T-stat value) | 50 | 61 | 80 | 70 |
| KS test of MFCC (T-stat value) | 50 | 64 | 70 | 90 |
| Lilliefors test of MFCC (T-stat value) | 73 | 71 | 70 | 70 |
| Chi Sq Test of Mfcc (p- value) | 45 | 68 | 60 | 80 |
| KS test of MFCC (p-value) | 68 | 68 | 40 | 50 |
| Lilliefors test of MFCC (p- value) | 73 | 64 | 60 | 80 |
| NGS + Lilliefors test p value | 68 | 64 | 80 | 60 |

*Iphone* Trained Model tested on *Android* Dataset:

SET 1

| EPOCH LENGTH = 15 | Training Set Patient | | Test Set Patient | |
|---|---|---|---|---|
| | Sensitivity | Specificity | Sensitivity | Specificity |
| NGS of MFCC | **64** | 75 | 50 | 50 |
| Chi Sq Test of Mfcc (T-stat value) | 64 | 54 | 30 | 70 |
| KS test of MFCC (T-stat value) | 52 | 89 | 40 | 90 |
| Lilliefors test of MFCC (T-stat value) | 56 | 82 | 60 | 70 |
| Chi Sq Test of Mfcc (p- value) | 60 | 75 | 70 | 60 |
| KS test of MFCC (p- value) | 52 | 89 | 40 | 90 |
| Lilliefors test of MFCC (p-value) | 76 | 68 | 30 | 90 |
| NGS + Lilliefors test p value | 64 | 61 | 60 | 50 |

| EPOCH LENGTH = 30 | Training Set Patient | | Test Set Patient | |
|---|---|---|---|---|
| | Sensitivity | Specificity | Sensitivity | Specificity |
| **NGS of MFCC** | 52 | 71 | 70 | 70 |
| **Chi Sq Test of Mfcc (T-stat value)** | 68 | 71 | 50 | 80 |
| **KS test of MFCC (T-stat value)** | 68 | 75 | 70 | 50 |
| **Lilliefors test of MFCC (T-stat value)** | 60 | 71 | 50 | 70 |
| **Chi Sq Test of Mfcc (p- value)** | 72 | 68 | 70 | 60 |
| **KS test of MFCC (p- value)** | 60 | 82 | 60 | 60 |
| **Lilliefors test of MFCC (p-value)** | 56 | 64 | 40 | 60 |
| **NGS + Lilliefors test p value** | 64 | 61 | 60 | 50 |

**SET 2**

| EPOCH LENGTH = 15 | Training Set Patient | | Test Set Patient | |
|---|---|---|---|---|
| | Sensitivity | Specificity | Sensitivity | Specificity |
| **NGS of MFCC** | 72 | 64 | 60 | 70 |
| **Chi Sq Test of Mfcc (T-stat value)** | 84 | 75 | 70 | 50 |
| **KS test of MFCC (T-stat value)** | 52 | 68 | 40 | 80 |
| **Lilliefors test of MFCC (T-stat value)** | 68 | 64 | 50 | 70 |
| **Chi Sq Test of Mfcc (p- value)** | 84 | 79 | 60 | 50 |
| **KS test of MFCC (p-value)** | 52 | 68 | 30 | 80 |
| **Lilliefors test of MFCC (p- value)** | 96 | 82 | 80 | 40 |
| **NGS + Lilliefors test p value** | 72 | 68 | 50 | 60 |

| EPOCH LENGTH = 30 | Training Set Patient | | Test Set Patient | |
|---|---|---|---|---|
| | Sensitivity | Specificity | Sensitivity | Specificity |
| **NGS of MFCC** | 68 | 89 | 60 | 80 |
| **Chi Sq Test of Mfcc (T-stat value)** | 84 | 79 | 60 | 50 |
| **KS test of MFCC (T-stat value)** | 60 | 68 | 50 | 80 |
| **Lilliefors test of MFCC (T-stat value)** | 84 | 79 | 70 | 50 |
| **Chi Sq Test of Mfcc (p- value)** | 84 | 79 | 60 | 40 |
| **KS test of MFCC (p-value)** | 32 | 54 | 20 | 70 |
| **Lilliefors test of MFCC (p- value)** | 76 | 68 | 90 | 60 |
| **NGS + Lilliefors test p value** | 68 | 89 | 60 | 80 |

**SET 3**

| EPOCH LENGTH = 15 | Training Set Patient | | Test Set Patient | |
|---|---|---|---|---|
| | Sensitivity | Specificity | Sensitivity | Specificity |
| **NGS of MFCC** | 52 | 64 | 80 | 60 |
| **Chi Sq Test of Mfcc (T-stat value)** | 72 | 54 | 60 | 70 |
| **KS test of MFCC (T-stat value)** | 24 | 54 | 50 | 70 |
| **Lilliefors test of MFCC (T-stat value)** | 52 | 71 | 90 | 70 |
| **Chi Sq Test of Mfcc (p- value)** | 44 | 71 | 70 | 50 |
| **KS test of MFCC (p-value)** | 16 | 54 | 50 | 70 |
| **Lilliefors test of MFCC (p- value)** | 56 | 82 | 50 | 60 |
| **NGS + Lilliefors test p value** | 68 | 71 | 80 | 60 |

| EPOCH LENGTH = 30 | Training Set Patient | | Test Set Patient | |
|---|---|---|---|---|
| | Sensitivity | Specificity | Sensitivity | Specificity |
| **NGS of MFCC** | 64 | 86 | 80 | 70 |
| **Chi Sq Test of Mfcc (T-stat value)** | 60 | 68 | 50 | 70 |
| **KS test of MFCC (T-stat value)** | 28 | 57 | 50 | 70 |
| **Lilliefors test of MFCC (T-stat value)** | 68 | 82 | 70 | 90 |
| **Chi Sq Test of Mfcc (p- value)** | 68 | 79 | 50 | 50 |
| **KS test of MFCC (p-value)** | 20 | 57 | 50 | 70 |
| **Lilliefors test of MFCC (p- value)** | 52 | 86 | 80 | 80 |
| **NGS + Lilliefors test p value** | 68 | 71 | 60 | 80 |

In general terms, a method according to an embodiment of an aspect of the present invention comprises a method for diagnosing a malady of a patient from sounds of the patient. The malady may be OSA or a respiratory disease such as pneumonia or some other impairment from normal health that results in changes to the sounds that a patient produces. The method includes the steps of initially making a digital recording of the sounds of the patient and that may be done with a contactless microphone as previously discussed. The digital recording is processed by one or more suitably programmed electronic processors to extract a multiplicity of features for sub-segments of each of a number epochs of the digital recording. Features comprising MFCCs have been discussed in detail but other features can also be used in other embodiments such as pitch, entropy, formants, NGS and higher-order spectra-based features. The features are suitably stored in an electronic data storage apparatus such as an electronic or magnetic storage device or server or network accessible storage. The method then involves operating the processors for determining deviation scores from a probability distribution for each epoch based on the extracted multiplicity of features which are retrieved from the storage. In the preferred embodiment the probability distribution that is used is the Gaussian distribution but other distributions can also be used and have been previously mentioned in the results tabled above. The one or more processors then generate a test vector derived from the deviation scores which is then applied to a pre-trained decision machine which is implemented by the processors or on another data network accessible hardware platform. The decision machine that has primarily been discussed is a LRM but other decisions machines such as artificial neural networks, Bayesian decision machines, support vector machines, might also be used.

Finally a diagnosis of malady on the basis of the output from the decision machine is presented on a display under control of the processors, for example to a clinician in order that suitable therapy can be applied to the patient if a malady has been found to be present. For example, therapy may involve administration of antibiotics (for patients suffering from pneumonia), application of controlled air pressure (for patients suffering from OSA) and other appropriate therapies based upon the diagnosis.

References:

[1] B. M. Altevogt and H. R. Colten, Sleep Disorders and Sleep Deprivation:: An Unmet Public Health Problem: National Academies Press, 2006.

[2] Wake Up Australia: the value of healthy sleep: Access Economics: Australian Sleep Association, 2004.

[3] T. Young, L. Evans, L. Finn, and M. Palta, "Estimation of the clinically diagnosed proportion of sleep apnea syndrome in middle-aged men and women," Sleep, vol. 20, pp. 705-706, 1997.

[4] K. E. Bloch, "Polysomnography: a systematic review," Technol Health Care, vol. 5, pp. 285-305, 1997.

[5] E. Lugaresi, S. Mondini, M. Zucconi, P. Montagna, and F. Cirignotta, "Staging of heavy snorers' disease. A proposal," Bulletin europeen de physiopathologie respiratoire, vol. 19, pp. 590-594, 1982.

[6] H. Michael, S. Andreas, B. Thomas, H. Beatrice, H. Werner, and K. Holger, "Analysed snoring sounds correlate to obstructive sleep disordered breathing," European Archives of Oto-Rhino-Laryngology, vol. 265, pp. 105-113, 2008.

[7] W. Whitelaw, "Characteristics of the snoring noise in patients with and without occlusive sleep apnea," Am Rev RespirDis, vol. 147, pp. 635-644, 1993.

[8] A. K. Ng, T. S. Koh, E. Baey, T. H. Lee, U. R. Abeyratne, and K. Puvanendran, "Could formant frequencies of snore signals be an alternative means for the diagnosis of obstructive sleep apnea?," Sleep medicine, vol. 9, pp. 894-898, 2008.

[9] T. Emoto, U. R. Abeyratne, M. Akutagawa, S. Konaka, and Y. Kinouchi, "High frequency region of the snore spectra carry important information on the disease of sleep apnoea," Journal of medical engineering & technology, vol. 35, pp. 425-431, 2011.

[10] A. Azarbarzin and Z. Moussavi, "Snoring sounds variability as a signature of obstructive sleep apnea," Medical engineering & physics, vol. 35, pp. 479-485, 2013.

[11] J. Solà-Soler, J. A. Fiz, J. Morera, and R. Jané, "Multiclass classification of subjects with sleep apnoea-hypopnoea syndrome through snoring analysis," Medical engineering & physics, vol. 34, pp. 1213-1220, 2012.

[12] N. Ben-Israel, A. Tarasiuk, and Y. Zigel, "Obstructive apnea hypopnea index estimation by analysis of nocturnal snoring signals in adults," Sleep, vol. 35, pp. 1299-305C, 2012.

[13] H. Alshaer, G. R. Fernie, E. Maki, and T. Douglas Bradley, "Validation of an automated algorithm for detecting apneas and hypopneas by acoustic analysis of breath sounds," Sleep medicine, vol. 14, pp. 562-571, 2013.

[14] U. Abeyratne, S. de Silva, C. Hukins, and B. Duce, "Obstructive sleep apnea screening by integrating snore feature classes," Physiological Measurement, vol. 34, p. 99, 2013.

[15] U. R. Abeyratne, A. S. Karunajeewa, and C. Hukins, "Mixed-phase modeling in snore sound analysis," Medical and Biological Engineering and Computing, vol. 45, pp. 791-806, 2007.

[16] A. S. Karunajeewa, U. R. Abeyratne, and C. Hukins, "Multi-feature snore sound analysis in obstructive sleep apnea-hypopnea syndrome," Physiological measurement, vol. 32, p. 83, 2011.

[17] N. Oliver and F. Flores-Mangas, "HealthGear: automatic sleep apnea detection and monitoring with a mobile phone," Journal of Communications, vol. 2, pp. 1-9, 2007.

[18] S. Alqassim, M. Ganesh, S. Khoja, M. Zaidi, F. Aloul, and A. Sagahyroon, "Sleep apnea monitoring using mobile phones," in e-Health Networking, Applications and Services (Healthcom), 2012 IEEE 14th International Conference on, 2012, pp. 443-446.

[19] T. Hao, G. Xing, and G. Zhou, "iSleep: unobtrusive sleep quality monitoring using smartphones," in Proceedings of the 11th ACM Conference on Embedded Networked Sensor Systems, 2013, p. 4.

[20] H. Nakano, K. Hirayama, Y. Sadamitsu, A. Toshimitsu, H. Fujita, S. Shin, et al., "Monitoring sound to quantify snoring and sleep apnea severity using a smartphone: proof of concept," Journal of clinical sleep medicine: JCSM: official publication of the American Academy of Sleep Medicine, vol. 10, pp. 73-78, 2014.

[21] D. Salomon, Data compression: the complete reference: Springer, 2004.

[22] K. Sayood, Introduction to data compression: Newnes, 2012.

[23] H. Teager and S. Teager, "Evidence for nonlinear sound production mechanisms in the vocal tract," in Speech production and speech modelling, ed: Springer, 1990, pp. 241-261.

[24] H. Ghaemmaghami, U. Abeyratne, and C. Hukins, "Normal probability testing of snore signals for diagnosis of obstructive sleep apnea," in Engineering in Medicine and Biology Society, 2009. EMBC 2009. Annual International Conference of the IEEE, 2009, pp. 5551-5554.

In compliance with the statute, the invention has been described in language more or less specific to structural or methodical features. The term "comprises" and its variations, such as "comprising" and "comprised of" is used throughout in an inclusive sense and not to the exclusion of any additional features. It is to be understood that the invention is not limited to specific features shown or described since the means herein described herein comprises preferred forms of putting the invention into effect. The invention is, therefore, claimed in any of its forms or modifications within the proper scope of the appended claims appropriately interpreted by those skilled in the art.

Throughout the specification and claims (if present), unless the context requires otherwise, the term "substantially" or "about" will be understood to not be limited to the value for the range qualified by the terms.

Any embodiment of the invention is meant to be illustrative only and is not meant to be limiting to the invention. Therefore, it should be appreciated that various other changes and modifications can be made to any embodiment described without departing from the scope of the invention.

**Claims**

1. A method of operating one or more electronic processors (3) to diagnose the presence of a malady of the respiratory system of a patient comprising:

   accessing with said processors (3) a digital audio signal (31) of sounds of the patient (39) in an electronic storage assembly;
   identifying a number of epochs of the digital audio signal;
   identifying a plurality of sub-segments for each of the epochs;
   for each sub-segment of each of the epochs determining an associated multiplicity of mel-frequency cepstral coefficients, MFCCs;
   determining deviation scores from a probability distribution for each of the epochs in respect of each of the multiplicity of MFCCs;
   forming a test vector for the patient based upon the deviations scores from the probability distribution of the MFCCs;
   applying the test vector to a pre-trained decision machine stored in said electronic storage assembly to thereby generate a malady signal indicating malady or non-malady for the patient; and
   controlling a display responsive to the one or more electronic processors (3) to display a message corresponding to the malady signal.

2. A method according to claim 1, wherein forming of the test vector based upon the deviations scores of the MFCCs includes applying a comparator to each of the deviation scores.

3. A method according to claim 2, including forming components of the test vector for each of the MFCCs by producing sums of outputs from the comparator.

4. A method according to claim 3, including:

   producing the sums of the outputs from the comparator for each MFCC over all of the epochs;
   averaging each of the sums of the outputs over all of the epochs; and
   reducing dimensionality of the test vector by removing all but a subset of components of the test vector previously adjudged to be statistically significant for production of the malady signal from the pre-trained decision machine.

5. A method according to any one of claims 1 to 4, including forming the test vector on the basis of the entire digital audio signal.

6. A method according to any one of claims 1 to 5, wherein the probability distribution is a Gaussian distribution and the deviation from a probability distribution score is a non-Gaussianity Score, NGS.

7. A method according to any one of claims 1 to 6, wherein the malady is Obstructive Sleep Apnea, OSA.

8. An apparatus (100) for diagnosing the presence of malady of the respiratory system of a patient comprising:

   a microphone (120);
   an audio interface including an analog-to-digital converter, ADC, (122) coupled to the microphone (120);
   an electronic storage assembly (124) coupled to the ADC (122) and arranged to store a digitized audio file of patient sounds from the audio interface;
   an epoch identification assembly (128) configured to process the digitized audio file and identify a number of epochs therein;

a sub-segment identification assembly (130) configured to process the digitized audio file and identify a plurality of sub-segments therein for each of the epochs;

a Mel-Frequency Cepstral Coefficient generator (132) that is responsive to the epoch identification assembly (128) and the sub-segment identification assembly (130) and arranged to process the digitized audio file to produce a multiplicity of mel-frequency cepstral coefficients, MFCCs, signals for each of the sub-segments;

a deviation from probability distribution score assembly (134) that is responsive to the Mel-Frequency Cepstral Coefficient generator (132) and which is arranged to process the MFCCs signals for each of the sub-segments to produce deviation from probability distribution scores for each of the MFCCs signals for each epoch;

a test-vector generator assembly (136) that is responsive to the deviation from probability distribution score assembly (134) and which is arranged to store a test vector for the patient in the electronic storage assembly (124);

a decision assembly (142) that is coupled to the at least one electronic processor and arranged to process the test vector to produce a malady diagnosis signal; and

a human-machine interface that is coupled to the decision assembly and arranged to present the malady diagnosis to a human.

9. An apparatus according to claim 8, wherein the malady is Obstructive Sleep Apnea, OSA.

10. A computer readable medium bearing tangible, non-transitory machine readable instructions for execution by one or more electronic microprocessors including instructions to perform the method of any of claims 1 to 7.


**Patentansprüche**

1. Verfahren zum Betreiben eines oder mehrerer elektronischer Prozessoren (3), um das Vorliegen einer Krankheit der Atemwege eines Patienten zu diagnostizieren, umfassend:

Zugreifen, mit den Prozessoren (3), auf ein digitales Audiosignal (31) von Geräuschen des Patienten (39) in einer elektronischen Speicherbaugruppe;

Identifizieren einer Anzahl von Epochen des digitalen Audiosignals;

Identifizieren einer Vielzahl von Untersegmenten für jede der Epochen;

für jedes Untersegment jeder der Epochen Bestimmen einer zugehörigen Mehrzahl von Mel-Frequenz-Cepstrum-Koeffizienten, MFCCs;

Bestimmen von Abweichungswerten aus einer Wahrscheinlichkeitsverteilung für jede der Epochen in Bezug auf jeden der Mehrzahl von MFCCs;

Bilden eines Testvektors für den Patienten basierend auf den Abweichungswerten aus der Wahrscheinlichkeitsverteilung der MFCCs;

Anwenden des Testvektors auf eine vorab trainierte Entscheidungsmaschine, die in der elektronischen Speicherbaugruppe gespeichert ist, um dadurch ein Krankheitssignal zu erzeugen, das angibt, ob der Patient krank ist oder nicht; und

Steuern einer Anzeige, die auf den einen oder die mehreren elektronischen Prozessoren (3) reagiert, um eine dem Krankheitssignal entsprechende Nachricht anzuzeigen.

2. Verfahren nach Anspruch 1, wobei das Bilden des Testvektors basierend auf den Abweichungswerten der MFCCs das Anwenden eines Vergleichers auf jeden der Abweichungswerte einschließt.

3. Verfahren nach Anspruch 2, das das Bilden von Komponenten des Testvektors für jeden der MFCCs durch Produzieren von Summen der Ausgaben aus dem Vergleicher einschließt.

4. Verfahren nach Anspruch 3, das einschließt:

Produzieren der Summen der Ausgaben aus dem Vergleicher für jeden MFCC über alle Epochen hinweg;

Mitteln jeder der Summen der Ausgaben über alle Epochen hinweg; und

Reduzieren von Dimensionalität des Testvektors durch Entfernen aller Komponenten des Testvektors bis auf eine Untermenge von Komponenten, die zuvor von der vorab trainierten Entscheidungsmaschine als statistisch signifikant für die Produktion des Krankheitssignals beurteilt wurden.

**5.** Verfahren nach einem der Ansprüche 1 bis 4, das das Bilden des Testvektors basierend auf dem gesamten digitalen Audiosignal einschließt.

**6.** Verfahren nach einem der Ansprüche 1 bis 5, wobei die Wahrscheinlichkeitsverteilung eine Gauß-Verteilung ist und die Abweichung von einem Wahrscheinlichkeitsverteilungswert ein Nicht-Gauß'scher Wert, NGS, ist.

**7.** Verfahren nach einem der Ansprüche 1 bis 6, wobei die Krankheit obstruktive Schlafapnoe, OSA, ist.

**8.** Einrichtung (100) zum Diagnostizieren des Vorliegens einer Krankheit der Atemwege eines Patienten, umfassend:

ein Mikrofon (120);
eine Audioschnittstelle, die einen Analog-Digital-Wandler, ADC, (122) einschließt, der mit dem Mikrofon (120) gekoppelt ist;
eine elektronische Speicherbaugruppe (124), die mit dem ADC (122) gekoppelt ist und eingerichtet ist, um eine digitalisierte Audiodatei mit Patientengeräuschen von der Audioschnittstelle zu speichern;
eine Epochenidentifizierungsbaugruppe (128), die konfiguriert ist, um die digitalisierte Audiodatei zu verarbeiten und eine Anzahl von Epochen darin zu identifizieren;
eine Untersegmentidentifizierungsbaugruppe (130), die konfiguriert ist, um die digitalisierte Audiodatei zu verarbeiten und für jede der Epochen eine Vielzahl von Untersegmenten darin zu identifizieren;
einen Mel-Frequenz-Cepstrum-Koeffizienten-Generator (132), der auf die Epochenidentifizierungsbaugruppe (128) und die Untersegmentidentifizierungsbaugruppe (130) reagiert und eingerichtet ist, um die digitalisierte Audiodatei zu verarbeiten, um eine Mehrzahl von Mel-Frequenz-Cepstrum-Koeffizienten-Signalen, MFCC-Signalen, für jedes der Untersegmente zu produzieren;
eine Baugruppe (134) für Abweichungen von Wahrscheinlichkeitsverteilungswerten, die auf den Mel-Frequenz-Cepstrum-Koeffizienten-Generator (132) reagiert und die eingerichtet ist, um die MFCCs-Signale für jedes der Untersegmente zu verarbeiten, um für jedes der MFCCs-Signale für jede Epoche Abweichungen von Wahrscheinlichkeitsverteilungswerten zu produzieren;
eine Testvektorgeneratorbaugruppe (136), die auf die Baugruppe (134) für Abweichungen von Wahrscheinlichkeitsverteilungswerten reagiert und die eingerichtet ist, um einen Testvektor für den Patienten in der elektronischen Speicherbaugruppe (124) zu speichern;
eine Entscheidungsbaugruppe (142), die mit dem mindestens einen elektronischen Prozessor gekoppelt ist und eingerichtet ist, um den Testvektor zu verarbeiten, um ein Krankheitsdiagnosesignal zu produzieren; und
eine Mensch-Maschine-Schnittstelle, die mit der Entscheidungsbaugruppe gekoppelt ist und eingerichtet ist, um einem Menschen die Krankheitsdiagnose zu präsentieren.

**9.** Einrichtung nach Anspruch 8, wobei die Krankheit obstruktive Schlafapnoe, OSA, ist.

**10.** Computerlesbares Medium, das physische, nicht flüchtige, maschinenlesbare Anweisungen für eine Ausführung durch einen oder mehrere elektronische Mikroprozessoren, einschließlich Anweisungen, um das Verfahren nach einem der Ansprüche 1 bis 7 durchzuführen, trägt.

## Revendications

**1.** Procédé de fonctionnement d'un ou plusieurs processeurs électroniques (3) pour diagnostiquer la présence d'une maladie du système respiratoire d'un patient comprenant :

l'accès avec lesdits processeurs (3) à un signal audio numérique (31) de sons du patient (39) dans un ensemble de stockage électronique ;
l'identification d'un nombre d'époques du signal audio numérique ;
l'identification d'une pluralité de sous-segments pour chacune des époques ;
pour chaque sous-segment de chacune des époques la détermination d'une multiplicité associée de coefficients cepstraux de fréquence Mel, MFCC ;
la détermination de scores de déviation par rapport à une distribution de probabilité pour chacune des époques par rapport à chacun parmi la multiplicité de MFCC ;
la formation d'un vecteur de test pour le patient en fonction des scores de déviation par rapport à la distribution de probabilité des MFCC ;
l'application du vecteur de test à une machine de décision préentraînée stockée dans ledit ensemble de stockage électronique pour générer de ce fait un signal de maladie indiquant une maladie ou non-maladie pour le patient ; et la commande d'un affichage en réponse au ou aux processeurs électroniques (3) pour afficher un message correspondant au signal de maladie.

2. Procédé selon la revendication 1, dans lequel la formation du vecteur de test en fonction des scores de déviation des MFCC comporte l'application d'un comparateur à chacun des scores de déviation.

3. Procédé selon la revendication 2, comportant la formation de composants du vecteur de test pour chacun des MFCC en produisant des sommes de sorties provenant du comparateur.

4. Procédé selon la revendication 3, comportant :

   la production des sommes des sorties provenant du comparateur pour chaque MFCC sur toutes les époques ;
   le moyennage de chacune des sommes des sorties sur toutes les époques ; et
   la réduction de dimensionnalité du vecteur de test en retirant la totalité à l'exception d'un sous-ensemble de composants du vecteur de test précédemment jugés comme étant statistiquement significatifs pour la production du signal de maladie à partir de la machine de décision préentraînée.

5. Procédé selon l'une quelconque des revendications 1 à 4, comportant la formation du vecteur de test en fonction du signal audio numérique entier.

6. Procédé selon l'une quelconque des revendications 1 à 5, dans lequel la distribution de probabilité est une distribution gaussienne et le score de déviation par rapport à une distribution de probabilité est un score de non-gaussianité, NGS.

7. Procédé selon l'une quelconque des revendications 1 à 6, dans lequel la maladie est une apnée obstructive du sommeil, OSA.

8. Appareil (100) permettant de diagnostiquer la présence d'une maladie du système respiratoire d'un patient comprenant :

   un microphone (120) ;
   une interface audio comportant un convertisseur analogique vers numérique, ADC, (122) couplé au microphone (120) ;
   un ensemble de stockage électronique (124) couplé à l'ADC (122) et agencé pour stocker un fichier audio numérisé de sons de patient provenant de l'interface audio ;
   un ensemble d'identification d'époque (128) configuré pour traiter le fichier audio numérisé et identifier un nombre d'époques à l'intérieur de celui-ci ;
   un ensemble d'identification de sous-segments (130) configuré pour traiter le fichier audio numérisé et identifier une pluralité de sous-segments à l'intérieur de celui-ci pour chacune des époques ;
   un générateur de coefficient cepstral de fréquence Mel (132) qui réagit à l'ensemble d'identification d'époque (128) et à l'ensemble d'identification de sous-segments (130) et est agencé pour traiter le fichier audio numérisé pour produire une multiplicité de signaux de coefficients cepstraux de fréquence Mel, MFCC, pour chacun des sous-segments ;
   un ensemble de score de déviation par rapport à une distribution de probabilité (134) qui réagit au générateur de coefficient cepstral de fréquence Mel (132) et qui est agencé pour traiter les signaux de MFCC pour chacun des sous-segments pour produire des scores de déviation par rapport à une distribution de probabilité pour chacun des signaux de MFCC pour chaque époque ;
   un ensemble générateur de vecteur de test (136) qui réagit à l'ensemble de score de déviation par rapport à une distribution de probabilité (134) et qui est agencé pour stocker un vecteur de test pour le patient dans l'ensemble de stockage électronique (124) ;
   un ensemble de décision (142) qui est couplé à l'au moins un processeur électronique et agencé pour traiter le vecteur de test pour produire un signal de diagnostic de maladie ; et
   une interface humain-machine qui est couplée à l'ensemble de décision et agencée pour présenter le diagnostic de maladie à un humain.

9. Appareil selon la revendication 8, dans lequel la maladie est une apnée obstructive du sommeil, OSA.

10. Support lisible par ordinateur portant des instructions tangibles non transitoires lisibles par machine pour exécution par un ou plusieurs microprocesseurs électroniques comportant des instructions pour mettre en oeuvre le procédé selon l'une quelconque des revendications 1 à 7.

FIG. 1

59

1

11

*Sleep Apnea Fast*

Record

Pat ID:   12032014-32

Timeout (hrs)   10   +

44100 Hz

61

1

11

*Sleep Apnea Fast*

Recording...

Press 'OK' to stop recording OR Press 'Cancel' to cancel all processes

OK   Cancel

FIG. 2

FIG. 3

Nocturnal breathing sound Signal, x
41

Segment x into non-overlapping blocks of size $k_1$ = [10,15,20,30] seconds, x(i) where i = 1,2,...,N.
43

Resample x(i) using Fs = [44100; 22050; 11025; 8000; 6000; 4000; 2000; 1000; 500; 200]
45

Filter X(i) using band pass filter [LCF = 20 Hz; HCF = Fs/2]
47

Pre-emphasis (alpha = $e^{2 \times \pi \times 20 \times 1/Fs}$) filter to get Y(i)
48

Sub-segment Y(i) using a non-overlapping rectangular window of length $k_2$ = 100ms, Y(i,j) where j=1,2,...,J.
49

(A)

FIG. 4

(A) → Compute 12 MFCC components
_51_ from each j$^{th}$ sub-segment. $Z_c(i,j)$

↓

Compute non-gaussianity score.
$\xi_c(i)$. of a MFCC component values
_53_ in an epoch.

↓

Check
_55_
$\xi_c(i) > \eta$

Yes ↓     No ↓

Define $L_c(i) = 1$
_57_

Define $L_c(i) = 0$
_59_

↓     ↓

Compute MFCC-Index
Feature Vector

$$\Psi_c = \frac{\sum_{i=1}^{N} L_c(i)}{N}$$

_61_

FIG. 5

↓

_63_ Form $\Psi_{\text{sc-test}}$ from $\Psi$

↓

_65_ Process $\Psi_{\text{sc-test}}$ with pre-trained LRM

↓

Operate screen to display OSA diagnosis
_67_

<u>FIG. 6</u>

<u>FIG. 7</u>

<u>FIG. 8</u>

## FIG. 9

## FIG. 10

FIG. 11

FIG. 12

FIG. 13

FIG. 14

63

1

11

*Sleep Apnea Fast*

**Pat ID:** 123032014-32

**Recording time (hrs):** 10

**Fs:** 44100Hz

**Audio File Type:** WAV

**Diagnosis:**

65

OSA Present

OK

FIG. 14A

FIG. 15

FIG. 16

FIG. 17

FIG. 18

100

122  120

ADC

121

*x*

Data Storage
Assembly 124

*x*

Pre-emphasis
126

*y*

System Clock
123

Epoch ID
Assembly 128

$y(i)$

$y(i)$

Sub-segment ID
Assembly 130

$y(i)$

NGS 134
Calculator

$\xi_c(i)$

$y(i,j)$

MFCC Generator
132

$z_c(i,j)$

Comparator
136

$L_c(i)$

Decision
Machine
(LRM) 142

$\Psi_{\text{sc-test}}$

Component
Reduction 140

$\Psi_c$

Sum and
Average 138

OSA /
non-OSA

Diagnostic
Display
146

FIG. 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2018903933
- US 2012004749 A1
- US 10098569 B2


**Non-patent literature cited in the description**

- **H. GHAEMMAGHAMI ; U. ABEYRATNE ; C. HUKINS.** Normal probability testing of snore signals for diagnosis of obstructive sleep apnea. *Engineering in Medicine and Biology Society, 2009. EMBC 2009. Annual International Conference of the IEEE,* 2009, 5551-5554
- Sleep Disorders and Sleep Deprivation. **B. M. ALTEVOGT ; H. R. COLTEN.** An Unmet Public Health Problem. National Academies Press, 2006
- Wake Up Australia: the value of healthy sleep. *Access Economics: Australian Sleep Association,* 2004
- **T. YOUNG ; L. EVANS ; L. FINN ; M. PALTA.** Estimation of the clinically diagnosed proportion of sleep apnea syndrome in middle-aged men and women. *Sleep,* 1997, vol. 20, 705-706
- **K. E. BLOCH.** Polysomnography: a systematic review. *Technol Health Care,* 1997, vol. 5, 285-305
- **E. LUGARESI ; S. MONDINI ; M. ZUCCONI ; P. MONTAGNA ; F. CIRIGNOTTA.** Staging of heavy snorers' disease. A proposal. *Bulletin europeen de physiopathologie respiratoire,* 1982, vol. 19, 590-594
- **H. MICHAEL ; S. ANDREAS ; B. THOMAS ; H. BEATRICE ; H. WERNER ; K. HOLGER.** Analysed snoring sounds correlate to obstructive sleep disordered breathing. *European Archives of Oto-Rhino-Laryngology,* 2008, vol. 265, 105-113
- **W. WHITELAW.** Characteristics of the snoring noise in patients with and without occlusive sleep apnea. *Am Rev RespirDis,* 1993, vol. 147, 635-644
- **A. K. NG ; T. S. KOH ; E. BAEY ; T. H. LEE ; U. R. ABEYRATNE ; K. PUVANENDRAN.** Could formant frequencies of snore signals be an alternative means for the diagnosis of obstructive sleep apnea. *Sleep medicine,* 2008, vol. 9, 894-898
- **T. EMOTO ; U. R. ABEYRATNE ; M. AKUTAGAWA ; S. KONAKA ; Y. KINOUCHI.** High frequency region of the snore spectra carry important information on the disease of sleep apnoea. *Journal of medical engineering & technology,* 2011, vol. 35, 425-431
- **A. AZARBARZIN ; Z. MOUSSAVI.** Snoring sounds variability as a signature of obstructive sleep apnea. *Medical engineering & physics,* 2013, vol. 35, 479-485
- **J. SOLÀ-SOLER ; J. A. FIZ ; J. MORERA ; R. JANÉ.** Multiclass classification of subjects with sleep apnoea-hypopnoea syndrome through snoring analysis. *Medical engineering & physics,* 2012, vol. 34, 1213-1220
- **N. BEN-ISRAEL ; A. TARASIUK ; Y. ZIGEL.** Obstructive apnea hypopnea index estimation by analysis of nocturnal snoring signals in adults. *Sleep,* 2012, vol. 35, 1299-305C
- **H. ALSHAER ; G. R. FERNIE ; E. MAKI ; T. DOUGLAS BRADLEY.** Validation of an automated algorithm for detecting apneas and hypopneas by acoustic analysis of breath sounds. *Sleep medicine,* 2013, vol. 14, 562-571
- **U. ABEYRATNE ; S. DE SILVA ; C. HUKINS ; B. DUCE.** Obstructive sleep apnea screening by integrating snore feature classes. *Physiological Measurement,* 2013, vol. 34, 99
- **U. R. ABEYRATNE ; A. S. KARUNAJEEWA ; C. HUKINS.** Mixed-phase modeling in snore sound analysis. *Medical and Biological Engineering and Computing,* 2007, vol. 45, 791-806
- **A. S. KARUNAJEEWA ; U. R. ABEYRATNE ; C. HUKINS.** Multi-feature snore sound analysis in obstructive sleep apnea-hypopnea syndrome. *Physiological measurement,* 2011, vol. 32, 83
- **N. OLIVER ; F. FLORES-MANGAS.** HealthGear: automatic sleep apnea detection and monitoring with a mobile phone. *Journal of Communications,* 2007, vol. 2, 1-9

- **S. ALQASSIM ; M. GANESH ; S. KHOJA ; M. ZAIDI ; F. ALOUL ; A. SAGAHYROON.** Sleep apnea monitoring using mobile phones. *e-Health Networking, Applications and Services (Healthcom), 2012 IEEE 14th International Conference on,* 2012, 443-446
- **T. HAO ; G. XING ; G. ZHOU.** iSleep: unobtrusive sleep quality monitoring using smartphones. *Proceedings of the 11th ACM Conference on Embedded Networked Sensor Systems,* 2013, 4
- **H. NAKANO ; K. HIRAYAMA ; Y. SADAMITSU ; A. TOSHIMITSU ; H. FUJITA ; S. SHIN et al.** Monitoring sound to quantify snoring and sleep apnea severity using a smartphone: proof of concept. *Journal of clinical sleep medicine: JCSM: official publication of the American Academy of Sleep Medicine,* 2014, vol. 10, 73-78
- **D. SALOMON.** Data compression: the complete reference. Springer, 2004
- **K. SAYOOD.** *Introduction to data compression: Newnes,* 2012
- Evidence for nonlinear sound production mechanisms in the vocal tract. **H. TEAGER ; S. TEAGER.** Speech production and speech modelling. Springer, 1990, 241-261